(19) Europäisches Patentamt
European Patent Office
Office européen des brevets

(11) Veröffentlichungsnummer: **0 195 963**
**A1**

(12) # EUROPÄISCHE PATENTANMELDUNG

(21) Anmeldenummer: **86102971.8**

(22) Anmeldetag: **06.03.86**

(51) Int. Cl.4: **C07D 205/08** , C07F 7/18 , C07F 9/65 , C07D 403/12 , C07D 487/04 , //C07C119/00,C07C154/00,(C07D487/04,209:00,205:00)

(30) Priorität: **19.03.85 DE 3509769**

(43) Veröffentlichungstag der Anmeldung: **01.10.86 Patentblatt 86/40**

(84) Benannte Vertragsstaaten: **AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Häbich, Dieter, Dr.**
**Krummacherstrasse 82**
**D-5600 Wuppertal 1(DE)**
Erfinder: **Hartwig, Wolfgang, Dr.**
**Pahlkestrasse 3**
**D-5600 Wuppertal 1(DE)**

(54) **Verfahren zur Herstellung von Carbapenemzwischenprodukten.**

(57) 4-[3-Carboxy-3-diazo-2-oxo-propylazetidin-2-one der allgemeinen Formel I

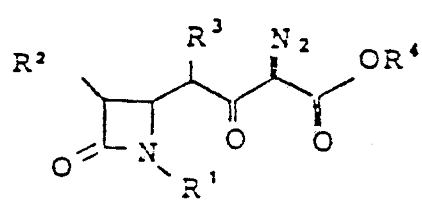

(I),

sind wichtige Zwischenprodukte zur Synthese von Carbapenemantibiotika. Sie werden durch Umsetzung von 4-Acetoxy-2-azetidinone der allgemeinen Formel II

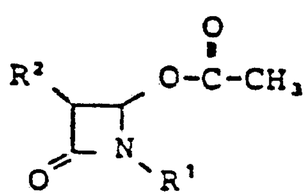

(II),

mit Verbindungen der allgemeinen Formel III

EP 0 195 963 A1

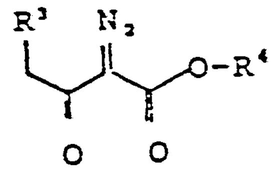

(III),

in einem inerten Lösungsmittel, in Gegenwert einer Base und eines Silylierungsmittels in einem Eintopfverfahren hergestellt.

Verfahren zur Herstellung von Carbapenemzwischenprodukten

Die Erfindung betrifft ein neues Verfahren zur Herstellung von teilweise bekannten 4-[3-Carboxy-3-diazo-2-oxopropyl]-azetidin-2-onen, die wichtige Zwischenprodukte zur Synthese von Carbapenemantibiotika sind.

Es ist bekannt, daß 4-Acetoxyazetidinone mit Trimethylsilylenolethern in Gegenwart von Lewissäuren zu den entsprechenden C-C verknüpften 4-[3-Carboxy-3-diazo-2-oxopropyl]azetidin-2-onen reagieren (vgl. EP 78026; J.D. Buynak et al., J. Chem. Soc., Chem. Commun. 1984, 948). Hierbei ist jeweils die separate und sehr aufwendige Herstellung der verwendeten Trimethylsilylenolether notwendig. Da bei deren Herstellung bisher metallorganische Basen wie n-Butyllithium oder Lithiumdiisopropylamid eingesetzt wurden, sind diese Herstellungsmethoden mit der wichtigsten und am häufigsten verwendeten Carbapenemesterschutzgruppe, dem 4-Nitrobenzylester, nicht vereinbar. Außerdem wurden bei der Verknüpfung von Diazoenolethern bisher als Lewis-Säuren lediglich Zink-(II)-halogenide eingesetzt. Solche heterogene Reaktionen erfordern zum Teil erhöhte Reaktionstemperaturen sowie lange Reaktionszeiten (vgl. EP 78 026; J.D. Buynak, J. Chem. Soc., Chem. Commun. 1984, 984; P.J. Reider et al., Tetrahedron Lett. 1982, 379), was sich bekanntlich wegen der Empfindlichkeit der Produkte sehr nachteilig auswirken kann.

Außerdem ist bekannt, daß das Gelingen der Reaktion in den meisten Fällen den vorherigen und separaten Einbau einer N-Schutzgruppe erforderlich macht (vgl. M. Shiozaki et al., J. Org. Chem. 39, 2399 (1983); M. Shiozaki et al., Tetrahedron 40 , 1795 (1984) und Tetrahedron Lett. 1984, 2793; A.G.M. Barret J. Org. Chem. 49, 1679 (1984)).

Weiterhin ist bekannt, daß die direkte Umsetzung von 4-Acetoxyazetidinonen mit Lithiumenolaten schlechte Ausbeuten liefert und ebenfalls nur mit gegen Metallorganica unempfindlichen Estern durchführbar ist (vgl. T. Kametani et al., Heterocycles 14, 1967 (1980) ; T. Kametani et al., J. Chem. Soc., Perkin I 1981, 2228).

Es wurde nun gefunden, daß man 4-[3-Carboxy-3-diazo-2-oxo-propyl]-azetidin-2-one der allgemeinen Formel I

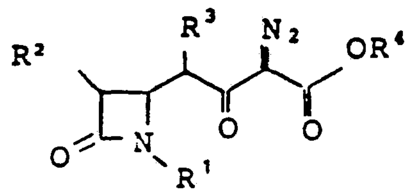

(I),

in welcher

$R^1$ -für Wasserstoff oder

-für eine Aminoschutzgruppe steht,

$R^2$ -für Wasserstoff,

-für gegebenenfalls substituiertes Alkyl,

-für gegebenenfalls substituiertes Aryl,

-für gegebenenfalls substituiertes Heterocyclyl,

-für Cyano, Azido, Halogen, oder

-für $NHR^1$ steht, wobei $R^1$ die oben angegebene Bedeutung hat,

$R^3$ -für Wasserstoff,

-für gegebenenfalls substituiertes Alkyl,

-für gegebenenfalls substituiertes Aryl,

-für gegebenenfalls substituiertes Heterocyclyl,

-für Halogen, Cyano, Cyanato, Azido, Nitro oder =N-Phenyl,

-für $-COR^5$, $-CO_2R^5$, $-CO-NR^5R^6$, $-C(=NR^5)NR^5R^6$, $-CSNR^5R^6$

-für $-NR^5R^6$, $-NR^5-COR^6$, $-NR^5-CO_2R^7$, $-NR^6-CR^5(=NR^6)$, $-NR^6-CO-NR^5R^6$, $-NR^5-CS-NR^5R^6$ $-NR^5-C(=NR^6)-NR^5R^6$, $-NR^5-SO_2R^7$, oder

-für $OR^5$, $-OCOR^5$, $-OCO-NR^5R^6$, $-OSO_2R^7$, $OSO_3R^5$, $-OPO(OR^5)OR^6$, oder

-für $-SR^5$, $-S(O)_nR^7$ wobei n=1 oder 2 ist, $-SCOR^5$, $-SO_2OR^5$, $-SO_2NR^5R^6$, -SCN, $-SCONHR^5$, oder

-für $-PX(OR^5)OR^6$, $PX(NR^5R^6)_2$, $PX(OR^5)NR^5R^6$ wobei jeweils

$R^5$, $R^6$ -gleich oder verschieden sind und

-für Wasserstoff,

-für gegebenenfalls substituiertes Alkyl,

-für gegebenenfalls substituiertes Aryl,

-für gegebenenfalls substituiertes Heterocyclyl steht, oder

- gegebenenfalls für eine Hydroxy-, Mercapto-, Amino-oder Carboxyschutzgruppe steht,

$R^7$ -die gleiche Bedeutung hat wie $R^5$, $R^6$, jedoch nicht für Wasserstoff oder eine Schutzgruppe steht,

und

X -für Sauerstoff oder Schwefel steht,

und

$R^4$ -für eine Carboxyschutzgruppe oder für einen in vivo spaltbaren Esterrest steht,

dadurch erhält, daß man 4-Acetoxy-2-azetidinone der allgemeinen Formel II

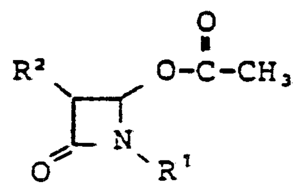 (II),

in welcher $R^1$ und $R^2$ die oben angegebene Bedeutung haben, mit Verbindungen der allgemeinen Formel III

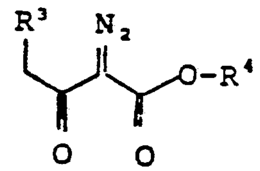 (III),

in welcher $R^3$ und $R^4$ die oben angegebene Bedeutung haben, in einem inerten Lösungsmittel, in Gegenwart einer Base und eines Silylierungsmittels in einem Eintopfverfahren umsetzt.

Es ist als äußerst überraschend zu bezeichnen, daß man die Verbindung der Formel I nach dem erfindungsgemäßen Verfahren in guten Ausbeuten erhält. Aufgrund des Standes der Technik war nicht zu erwarten, daß man die Verbindung der allgemeinen Formel I durch das erfindungsgemäße Eintopfverfahren in homogener Lösung herstellen kann.

Das erfindungsgemäße Verfahren hat außer einfacher Durchführung und milden Bedingungen den Vorteil, daß die aufwendige separate Herstellung der hydrolyseempfindlichen Vorstufen entfällt. Außerdem erlaubt das neue Verfahren auch die Verwendung der wichtigen Nitrobenzyl-Schutzgruppen. Das eingesetzte Silylierungsmittel wirkt gleichzeitig als Katalysator, was den Einsatz von weiteren Lewissäuren unnötig macht. Weiterhin ist das Verfahren auch anwendbar zur Herstellung von solchen Verbindungen I, in denen der Rest $R^3$ ein über ein Heteroatom gebundener Substituent ist (z.B. $-OCH_3$).

Die nach dem erfindungsgemäßen Verfahren herstellbaren Azetidinone sind durch die Formel (I) allgemein definiert. In dieser Formel stehen vorzugsweise

$R^1$ -für Wasserstoff oder

-für eine Aminoschutzgruppe,

$R^2$ -für Wasserstoff,

-für Fluor, Chlor, Brom oder Azido,

-für Phenyl,

-für $NHR^1$, wobei $R^1$ die oben angegebene Bedeutung hat,

-für einen Rest der Formel

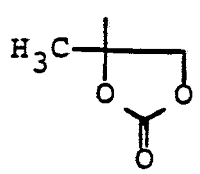 , 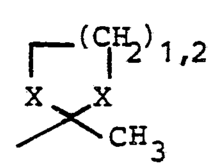 oder 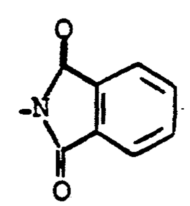 ,

wobei X Schwefel oder Sauerstoff bedeutet oder

-für geradkettiges, verzweigtes oder cyclisches, gesättigtes oder ungesättigtes Alkyl mit bis zu 6 C-Atomen, das gegebenenfalls substituiert ist durch Fluor, Chlor oder die Gruppe $OR^8$, wobei

$R^8$ -für Wasserstoff,

-für Trimethylsilyl, Triethylsilyl, Triisopropylsilyl, tert.-Butyldimethylsilyl, Triphenylsilyl, Trimethylsilylethoxycarbonyl,

-für Benzyl, Benzyloxycarbonyl, 2-bzw. 4-Nitrobenzyl, 2-bzw. 4-Nitrobenzyloxycarbonyl, tert.-Butyloxycarbonyl, Allyloxycarbonyl, 4-Methoxybenzyl, 4-Methoxybenzyloxycarbonyl, oder

-für Formyl, Acetyl, Trichloracetyl, Trichlorethoxycarbonyl, 2,4-Dimethoxybenzyloxycarbonyl, Methoxymethyl, Methylthiomethyl, Methoxyethoxymethyl, [2,(Trimethylsilyl)ethoxy]-methyl, 2-(Methylthiomethoxy)ethoxycarbonyl, Tetrahydropyranyl, oder Benzoyl steht,

$R^3$ -für Wasserstoff,

-für gegebenenfalls durch Chlor, Nitro, Methyl, Methoxy, Methylthio, Trifluormethyl oder durch (gegebenenfalls geschütztes) Hydroxy substituiertes Phenyl,

-für Fluor, Chlor, Brom, Cyano, Cyanato oder Azido,

-für gegebenenfalls durch Methyl, Amino(gegebenenfalls geschützt), Fluor, Chlor, Trifluormethyl oder Hydroxy substituiertes Thienyl, Furyl, Imidazolyl, Thiazolyl, Thiadiazolyl, Triazolyl, Tetrazolyl, Pyridyl, Piperazinyl oder Pyrimidyl,

-für S-Heterocyclyl, wobei Heterocyclyl die Bedeutung von gegebenenfalls durch Methyl, Amino, Hydroxy oder Acetylamino substituiertes Triazolyl, Tetrazolyl, Thiadiazolyl, Furyl, Thienyl, Pyridyl oder Pyrimidyl hat,

-für $-COR^5$, $-CO_2R^5$, $-CONHR^5$, Amidino,

-für $-NR^5R^6$, Ureido, Guanidino,

-für $-OR^5$, $-OCOR^5$, $-OSO_2R^7$, $-OPO(OR^5)_2$,

-für $-SR^5$, $-S(O)_nR^7$ mit n = 1,2, $-SCOR^5$, $-SCONHR^5$, $-SO_2OR^5$, $-SO_2NHR^5$, -SCN oder

-für $-PO(OR^5)_2$, $-PO(NR^5R^6)_2$,

wobei jeweils

$R^5$, $R^6$ -gleich oder verschieden sind und

-für Wasserstoff.

-für geradkettiges, verzweigtes oder cyclisches, gesättigtes oder ungesättigtes Alkyl mit bis zu 6 C-Atomen steht, das gegebenenfalls durch Phenyl oder ein oder mehrere Fluor substituiert ist,

- für gegebenenfalls durch Nitro oder Methoxy substituiertes Phenyl,

-für Furyl, Thienyl oder Pyridyl, oder

-für eine Hydroxy-, Mercapto-, Amino-oder Carboxyschutzgruppe steht,

$R^7$ -die gleiche Bedeutung hat wie $R^5$, $R^6$, jedoch nicht für Wasserstoff oder eine Schutzgruppe steht,

oder

-für geradkettiges, verzweigtes oder cyclisches, gesättigtes oder ungesättigtes Alkyl mit bis zu 6 C-Atomen, das gegebenenfalls substituiert ist durch Phenyl, Fluor, Chlor, Furyl, Thienyl, Pyridyl, Imidazolyl, Triazolyl, Tetrazolyl, Thiadiazolyl oder einen Rest der Formel

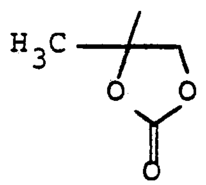

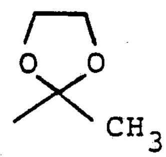

oder

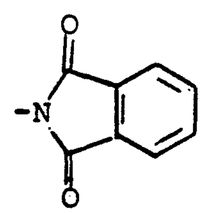

oder durch $-CO_2R^5$, $-CONHR^5$, Cyano, Amidino, $-NHR^5$, $-NHCOCH_3$, Guanidino, Azido, $-OR^5$, $-OCOR^5$, $-OSO_2R^7$, $-OPO(OR^5)_2$, $-SR^5$, $-SO_2R^7$, $-SCOR^5$, $-SO_2NHR^5$, $-PO(OR^5)_2$, $-PO(NR^5R^6)_2$, wobei $R^5$, $R^6$, $R^7$, die oben angegebene Bedeutung haben,

und

$R^4$ -für eine Carboxyschutzgruppe oder für einen in vivo spaltbaren Esterrest.

Das erfindungsgemäße Eintopfverfahren zur Herstellung von Verbindungen der allgemeinen Formel I faßt mehrere einzelne Reaktionsschritte zusammen.

Verwendet man als Ausgangsprodukte

a) (3R,4R)-4-Acetoxy-3-[1(R)-1-tert.butyldimethylsilyloxyethyl]azetidin-2-on ($R^1$ ind II = H) und Allyl-2-diazo-3-oxo-pentanoat oder

b) (3R,4R)-4-Acetoxy-3-[1(R)-1-tert.butyldimethylsilyloxyethyl]-1-(4-methoxyphenyl)azetidin-2-on ($R^1$ in II ≠ H) und 4-Nitrobenzyl-2-diazo-3-oxo-butanoat.

so läßt sich der Reaktionsablauf durch folgende Schemata verdeutlichen:

a)

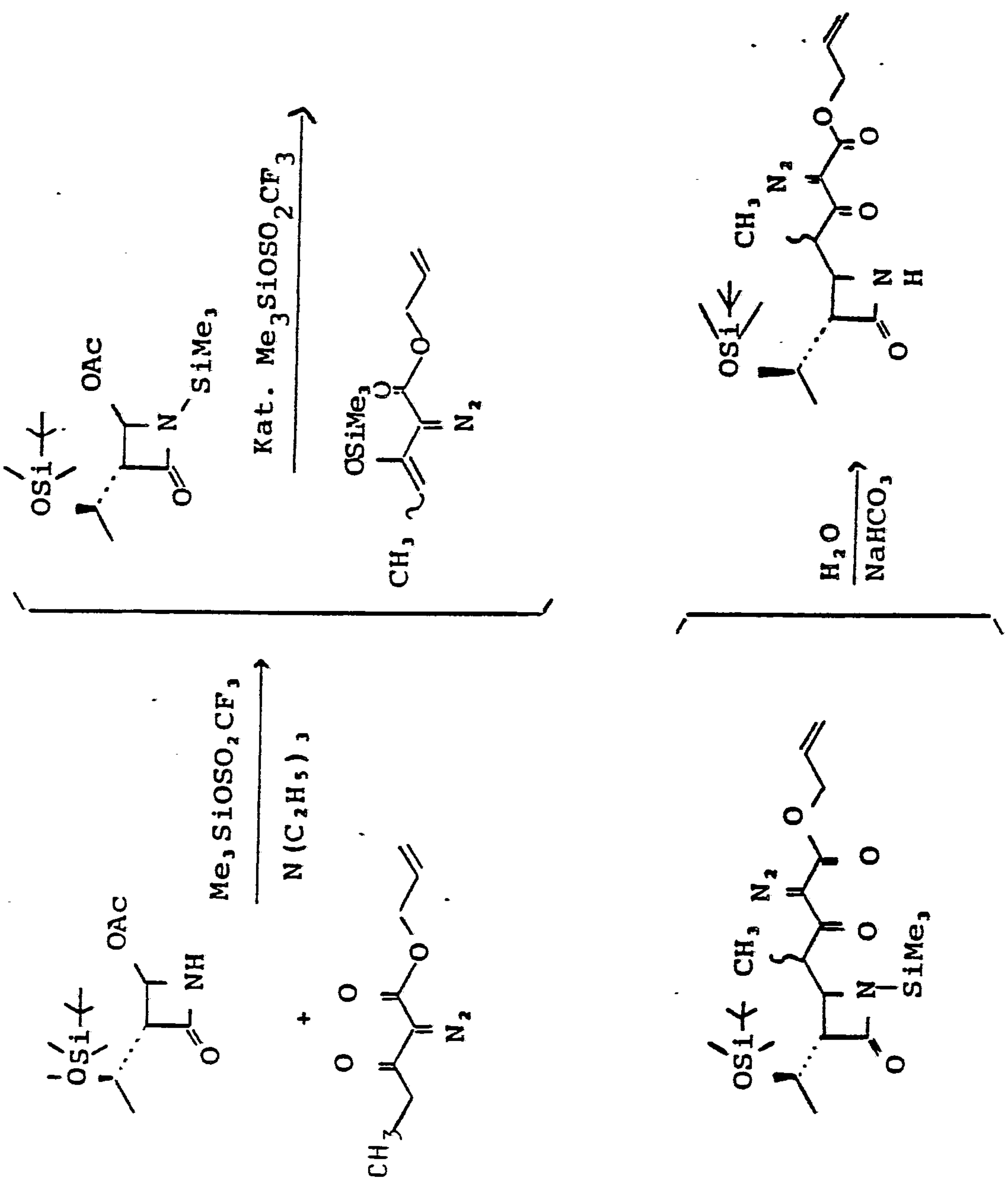

b)

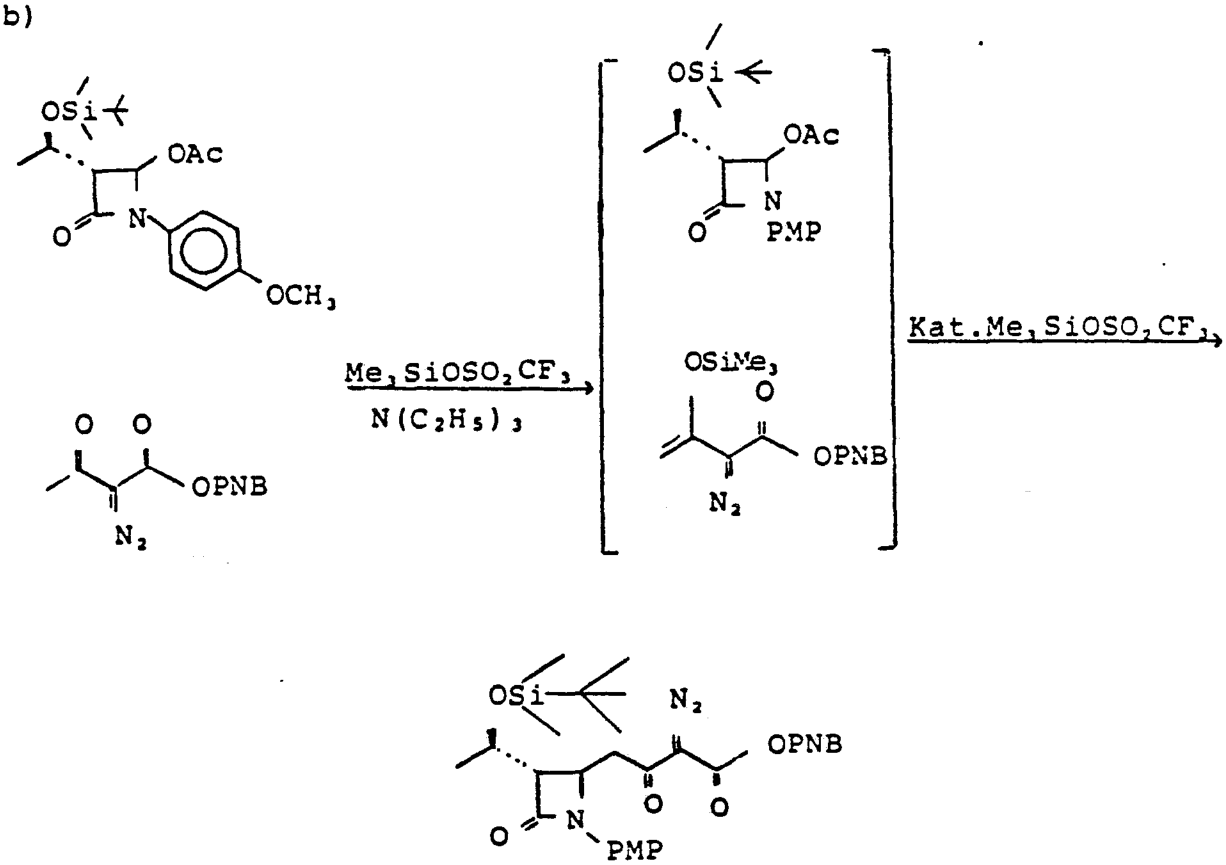


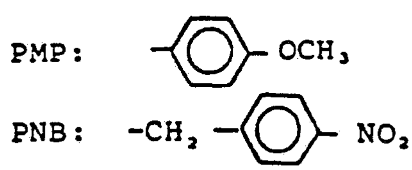

In den Verbindungen der allgemeinen Formel I, II bzw. III stehen $R^2$, $R^3$, $R^5$, $R^6$ und $R^7$ in der Bedeutung von gegebenenfalls substituiertem Alkyl für geradkettiges, verzweigte oder cyclisches, gesättigtes oder ungesättigtes Alkyl bis zu 10 C-Atomen, bevorzugt bis 6 C-Atomen. Als Substituenten kommen hierbei in Frage:

a) Phenyl, das wiederum mehrfach, bevorzugt bis zu zweifach substituiert sein kann durch Methyl, Methoxy, Methylthio, Trifluormethyl, Trifluormethoxy, Trifluormethylthio, Nitro, Cyano, jeweils gegebenenfalls geschütztes Hydroxy, Mercapto oder Amino, Dimethylamino, Carbamoyl, Amidino, Sulfo, Sulfamoyl oder Halogen, bevorzugt Fluor, Chlor oder Brom,

b) gesättigtes oder ungesättigtes 5-6-gliedriges, gegebenenfalls aneinander anelliertes Heterocyclyl mit 1-4 gleichen oder verschiedenen Heteroatomen aus der Gruppe O,S oder N, bevorzugt Thienyl, Furyl, Pyrrolyl, Imidazolyl, Pyrazolyl, Isothiazolyl, Thiazolyl, Oxazolyl, Isoxazolyl, Thiazolyl, Tetrazolyl, Thiadiazolyl, Pyridyl, Pyrazinyl, Pyrimidyl, Pyridazinyl, Indolyl, Chinolyl, Isochinolyl, Chinoxalyl, Chinazolyl, Purinyl, Pteridinyl, Pyrrolidinyl, Piperidinyl, Piperazinyl, Morpholinyl, Thiomorpholinyl, oder ein Rest der Formel

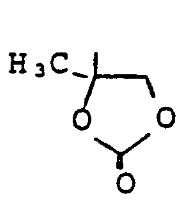 , 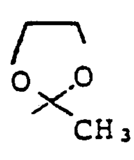 oder 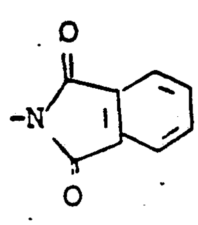 ,

wobei die Heterocyclen wiederum die gleichen Substituenten tragen können wie für Phenyl bereits angegeben,

c) Halogen, bevorzugt Fluor, Chlor, Brom

d) $-COR^9$, $-CO_2R^9$, $-CO-NR^9R^{10}$, $-C(=NR^{10})NR^9R^{10}$, -CN,

e) $-NR^9R^{10}$, $-NR^9-COR^{10}$, $-N(COR^9)(COR^{10})$, $-NR^9CO_2R^{11}$, $-NR^{10}-CR^9(=NR^{10})$, $-NR^{10}-CO-NR^9R^{10}$, $-NR^9-C(=NR^{10})-NR^9R^{10}$, $-NR^9-SO_2R^{11}$, $-N_3$,

f) $-OR^9$, $-OCOR^9$, $-OCO-NR^9R^{10}$, $-OSO_2R^{11}$, $-OSO_3R^9$, $-OPO(OR^9)OR^{10}$,

g) $-SR^9$, $-S(O)_nR^{11}$ wobei n = 1 oder 2 ist, $-SCOR^{11}$, $-SO_2OR^9$, $-SO_2NR^9R^{10}$, oder

h) $-PX(OR^9)OR^{10}$, $-PX(NR^9R^{10})_2$, $-PX(OR^9)NR^9R^{10}$, wobei jeweils

$R^9$, $R^{10}$ -gleich oder verschieden sind und

-für Wasserstoff,

-für geradkettiges, verzweigtes oder cyclisches gesättigtes oder ungesättigtes Alkyl bis $C_{10}$, bevorzugt bis $C_6$

steht,

-für Phenyl oder Benzyl,

-für Heterocyclyl oder Heterocyclylmethyl steht, wobei Heterocyclyl die unter b) angegebene Bedeutung hat, oder

-gegebenenfalls für eine Hydroxy-, Mercapto-, Amino-oder Carboxyschutzgruppe steht,

$R^{11}$ -die gleiche Bedeutung hat wie $R^9$, $R^{10}$, jedoch nicht für Wasserstoff oder eine Schutzgruppe steht,

und

X -für Sauerstoff oder Schwefel steht.

$R^2$, $R^3$, $R^5$, $R^6$, $R^7$ in der Bedeutung von gegebenenfalls substituiertem Aryl steht vorzugsweise für Phenyl, welches durch $C_1$-$C_4$-Alkyl (bevorzugt Methyl, Ethyl), $C_1$-$C_4$-Alkoxy - (bevorzugt Methoxy), $C_1$-$C_4$-Alkylthio (bevorzugt Methylthio), Trifluormethyl, Trifluromethoxy, Trifluormethylthio, Halogen - (bevorzugt Fluor, Chlor, Brom), Cyano, Nitro, Azido, Dimethylamino, Amino, Hydroxy, Mercapto (jeweils ggf. geschütztes), Sulfo, Sulfamoyl, Acetoxy oder Carbamoyl vorzugsweise einfach oder doppelt, gegebenenfalls auch dreifach substituiert sein kann.

$R^2$, $R^3$, $R^5$, $R^6$, $R^7$ in der Bedeutung von gegebenenfalls substituiertem Heterocyclyl steht bevorzugt für gesättigtes oder ungesättigtes, 5-6-gliedriges, gegebenenfalls aneinander anelliertes Heterocyclyl mit 1-4 gleichen oder verschiedenen Heteroatomen aus der Gruppe O, S, N, besonders bevorzugt für Thienyl, Furyl, Pyrrolyl, Imidazolyl, Pyrazolyl, Isothiazolyl, Thiazolyl, Tetrazolyl, Thiadiazolyl, Pyridyl, Pyrazinyl, Pyrimidyl, Pyridazinyl, Indolyl, Chinolyl, Isochinolyl, Chinoxalyl, Pyrrolidinyl, Piperidinyl, Piperazinyl, Benzothiazolyl, Benzimidazolyl, Purinyl, Pteridinyl, Morpholinyl, Thiomorpholinyl oder für einen Rest der Formel

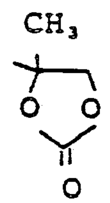
,
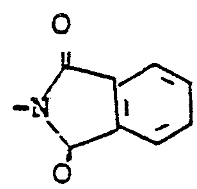
oder
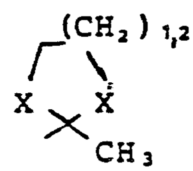

wobei als Substituenten von Heterocyclyl die für Aryl bereits angegebenen stehen und X für Schwefel oder Sauerstoff steht.

In $R^2$, $R^3$, $R^5$, $R^6$, $R^7$ steht Halogen bevorzugt für Fluor, Chlor oder Brom.

Wenn $R^1$ eine Aminoschutzgruppe ist, dann bevorzugt eine, die in der β-Lactam-Chemie üblich ist. Besonders bevorzugt zu nennen sind z.B. Vinyl, Allyl, tert.-Butyloxycarbonyl, Benzyl, Benzyloxycarbonyl, 2-Nitro-bzw. 4-Nitro-benzyl, 2-Nitro-bzw. 4-Nitro-benzyloxycarbonyl, 4-Methoxyphenyl, Formyl, Benzoyl, Acetyl, Chloracetyl, Trichloracetyl, Trifluoracetyl, Methyloxycarbonyl, Allyloxycarbonyl, Trimethyl-, Triethyl-bzw. Triphenylsilyl, tert.-Butyl-dimethylsilyl, 2,4-Dimethoxybenzyl, 2,4-Dimethoxybenzyloxycarbonyl, 4-Methoxybenzyl, 4-Methoxymethyloxyphenyl, Bis(4-Methoxyphenyl)methyl, tert.-Butoxycarbonylmethyl, Allyloxycarbonylmethyl, Methoxymethyl, Methylthiomethyl, Methoxyethoxymethyl, [2-(Trimethylsilyl)ethoxy]methyl, 2-(Methylthiomethoxy)-ethoxycarbonyl oder Tetrahydropyranyl.

Die gleiche Bedeutung haben auch die Begriffe Aminoschutzgruppe bzw. Hydroxy-bzw. Mercaptoschutzgruppe in den Resten $R^2$, $R^3$ und $R^5$-$R^{11}$.

$R^4$ sowie $R^5$, $R^6$, $R^9$, $R^{10}$ in der Bedeutung von Carboxyschutzgruppe steht für die in der β-Lactam-Chemie üblichen Schutzgruppen. Bevorzugt sind leicht abspaltbare Gruppen zu nennen wie Methyl, Ethyl, tert.-Butyl, Decyl, 2-Chlorethyl, 2,2,2-Trichlorethyl, Cyanoethyl, Di-bzw. Triphenylmethyl, Acetoxymethyl, Allyl, Benzyl, 4-Me thoxyphenyl, 4-Nitrobenzyl, 2-Nitrobenzyl, 2,4-Dimethoxybenzyl, Trimethylsilylethyl, Dimethyl-tert.-butylsilylethyl, Trimethylsilyl, Dimethyl-tert.-butylsilyl, Acetonyl, 1-Phenoxyethyl oder 2-Methyl-2-propenyl.

Die gleiche Bedeutung hat der Begriff Carboxyschutzgruppe in den Resten $R^2$, $R^3$, $R^5$, $R^6$, $R^9$ und $R^{10}$.

Steht $R^4$ für einen in vivo leicht spaltbaren Esterrest, so sind hiermit pharmazeutisch verträgliche Esterreste gemeint, die in vivo leicht zu freien Carboxylgruppen ($R^4$=H) hydrolisiert werden.

Solche Esterreste $R^4$ sind auf dem Penicillingebiet gut bekannt. In den meisten Fällen verbessern sie die Absorptionseigenschaften der β-Lactamverbindung. Außerdem sollte $R^4$ von solcher Art sein, daß er einer Verbindung der Formel (I) pharmazeutisch annehmbare Eigenschaften verleiht und bei Spaltung in vivo

pharmazeutisch annehmbare Fragmente freisetzt. Beispiele für solche Gruppen $R^4$ finden sich in der DE-OS 2 517 316. Bevorzugte Estergruppen $R^4$ sind die der folgenden Formeln

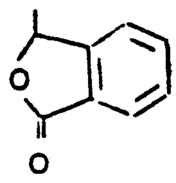 , 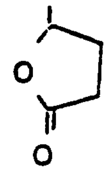 , 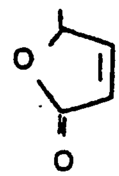 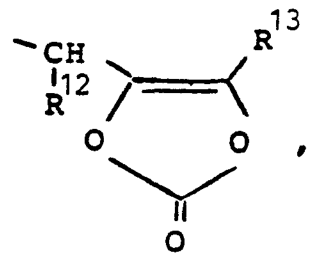 ,

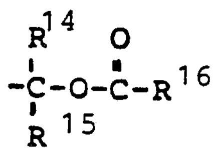 oder 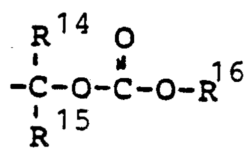 ,

worin

$R^{12}$, $R^{13}$ -gleich oder verschieden sind und

-für Wasserstoff,

-für Phenyl oder

-für $C_1$-$C_4$ Alkyl, bevorzugt Methyl steht.

$R^{14}$, $R^{15}$ -gleich oder verschieden sind und

-für Wasserstoff oder

-für $C_1$-$C_4$-Alkyl, bevorzugt Methyl, stehen und

$R^{16}$ -für $C_1$-$C_6$-Alkyl, bevorzugt $C_1$-$C_4$-Alkyl steht.

Einige Verbindungen der allgemeinen Formel I sind bereits bekannt, wurden jedoch nach anderen Verfahren hergestellt [vgl. Europäische Patentanmeldung 78 026; J.O. Buynak et al., J. Chem. Soc., Chem. Commun. 1984, 948: US Patente 4 444 685 und 4 400 323: D.H. Shih et al., Heterocycles 21 , 29 (1984)].

Neu und daher ebenfalls Gegenstand der Erfindung sind Verbindungen der Formel I, in welcher

$R^1$ -für Wasserstoff oder

-für eine Aminoschutzgruppe steht.

$R^2$ -für Wasserstoff,

-für Halogen,

-für Azido oder Phenyl,

-für $NHR^1$ steht, wobei $R^1$ die oben angegebene Bedeutung hat,

-für einen Rest der Formel

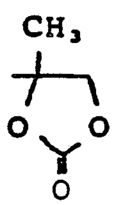 , 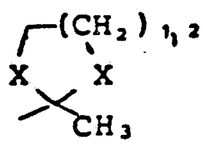 oder

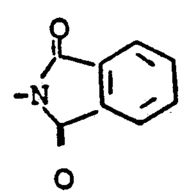 steht,

wobei X Schwefel oder Sauerstoff bedeutet, oder

-für geradkettiges, verzweigtes oder cyclisches, gesättigtes, oder ungesättigtes Alkyl (bis $C_6$) steht, das gegebenenfalls substituiert ist durch Fluor, Chlor oder die Gruppe $O$-$R^8$,

wobei

$R^8$ -für Wasserstoff,

-für Trimethylsilyl, Triethylsilyl, Triisopropylsilyl, tert.-Butyldimethylsilyl, Triphenylsilyl, Trimethylsilylethoxycarbonyl,

-für Benzyl, Benzyloxycarbonyl, 2-bzw. 4-Nitrobenzyl, 2-bzw. 4-Nitrobenzyloxycarbonyl, tert.-Butyloxycarbonyl, Allyloxycarbonyl, 4-Methoxybenzyl, 4-Methoxybenzyloxycarbonyl, oder

-für Formyl, Acetyl, Trichloracetyl, Trichlorethoxycarbonyl, 2,4-Dimethoxybenzyloxycarbonyl, Methoxy methyl, Methylthiomethyl, Methoxyethoxymethyl, [2,-(Trimethylsilyl)ethoxy]-methyl, 2-(Methylthiomethoxy)ethoxycarbonyl, Tetrahydropyranyl, oder Benzoyl steht,

$R^3$ -für gegebenenfalls durch Chlor, Nitro, Methyl, Methoxy, Methylthio, Trifluormethyl oder (ggfs. geschütztes) Hydroxy substituiertes Aryl ($C_6$, $C_{10}$) steht, oder

-für Halogen, Cyano, Azido, Cyanato, Trifluormethyl, oder Nitro

-für $-COR^{18}$, $-CONR^{18}R^{19}$, $-CSNR^{18}R^{19}$, oder

-für Allyloxycarbonyl oder 4-Nitrobenzyloxycarbonyl steht,

-für $-NR^{19}R^{18}$, Ureido, Guanidino, Amidino, $-NR^{18}SO_2R^{20}$,

-für $-OSO_2R^{20}$, $-OSO_3R^{18}$ oder $-OPO(OR^{19})OR^{18}$,

-für $-SR^{18}$, $-S(O)_nR^{20}$ mit n = 1,2, $-SCOR^{18}$, $-SCONHR^{18}$, $-SO_2OR^{18}$, $-SO_2NR^{19}R^{18}$, -SCN, oder

-für -S-Heterocyclyl steht, wobei Heterocyclyl die Bedeutung von gegebenenfalls durch Methyl, Amino, Hydroxy oder Acetylamino substituiertes Triazolyl, Tetrazolyl oder Thiadiazolyl, Furyl, Thienyl, Pyridyl oder Pyrimidyl hat,

- für $-PO(OR^{19})OR^{18}$ oder

-für

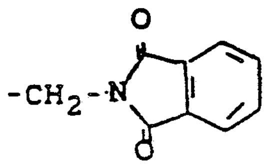

oder Benzyl steht,

wobei

$R^{18}$, $R^{19}$ -gleich oder verschieden sind und

-für Wasserstoff,

-für geradkettiges oder verzweigtes Alkyl (bis $C_6$) steht,

-für Phenyl oder Benzyl, (Phenyl gegebenenfalls durch Nitro, Methyl, oder Methoxy substituiert), oder

-für eine Hydroxy-, Mercapto-oder Aminoschutzgruppe steht und

$R^{20}$-für die gleiche Bedeutung wie $R^{18}$, $R^{19}$ hat, jedoch nicht für Wasserstoff oder eine Schutzgruppe steht,

und

$R^4$ -für Wasserstoff oder

-für Methyl, Ethyl, tert.Butyl, Decyl, 2-Chlorethyl, 2,2,2-Trichlorethyl, Cyanoethyl, Di-bzw. Triphenylmethyl, Acetoxymethyl, Allyl, Benzyl, 4-Methoxybenzyl, 2,4-Dimethoxybenzyl, tert.Butyl-dimethylsilyl, 1-Phenoxyethyl oder 2-Methyl-2-propenyl, 4-Nitrobenzyl, 2-Nitrobenzyl, Trimethylsilylethyl oder Dimethyl-tert.Butylsilylethyl, oder

-für einen Rest der Formel

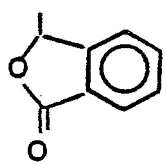 , 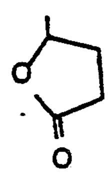 , 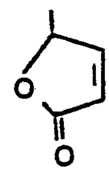 , 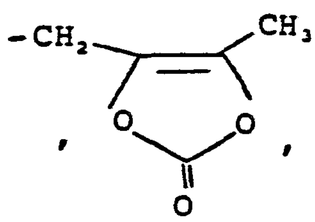 ,

$-CH_2-OCO-C(CH_3)_3$, $-CH(CH_3)OCOOC_2H_5$ oder $-CH_2OCOCH_3$ steht.

Bevorzugt sind die neuen Verbindungen der Formel I, in welchen

$R^1$ -für Wasserstoff oder

-für eine Aminoschutzgruppe steht,

$R^2$ -für Wasserstoff, Chlor, Brom, Azido,

-für $NHR^1$ steht, wobei $R^1$ die oben angegebene Bedeutung hat,

-für einen Rest der Formel

oder 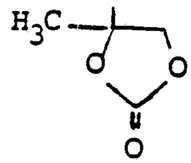 , 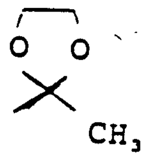

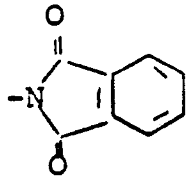

steht, oder

-für Methyl, Ethyl, i-Propyl, tert.Butyl, oder

-für -CH-OR[17], -C($CH_3$)$_2$-OR[17] oder

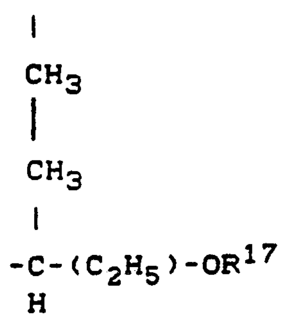 steht,

wobei

R[17] -für Wasserstoff, 3,4-Dimethoxyphenyl,

-für Trimethylsilyl, tert.Butyl-dimethylsilyl,

-für Benzyloxycarbonyl, 4-Nitrobenzyloxycarbonyl, tert.-Butoxycarbonyl, Allyloxycarbonyl, oder

-für Formyl oder Acetyl steht,

R[3] -für Phenyl, 3,4-Dimethoxyphenyl,

- für Chlor, Brom, Cyano, Cyanato, Azido oder $CF_3$ steht, oder

-für -COR[18], -CONR[19]R[18],

-für Allyloxycarbonyl oder 4-Nitrobenzyloxycarbonyl steht,

-für -NHR[18], Ureido, Guanidino, Amidino, -NHSO$_2$R[20],

-für -OSO$_2$R[20], -OPO(OR[19])OR[18]

-für-S-R[18], -SCOR[18], -SO$_2$OR[18], -SO$_2$NHR[18], -SCN, -SO$_2$R[20], -SCONH$_2$ steht, oder

-für -S-Pyridyl, -S-Pyrimidyl, -S-Methyltetrazolyl, -S-Thiadiazolyl, 2-Aminothiadiazolyl steht, oder

-für

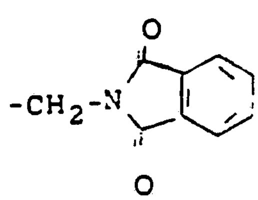

steht,

wobei

R[18], R[19] - gleich oder verschieden sind

-für Wasserstoff oder

-für geradkettiges oder verzweigtes Alkyl (bis $C_4$) steht,

-für Phenyl, 4-Methylphenyl oder Benzyl oder

-für eine Hydroxy-, Mercapto-oder Aminoschutzgruppe steht,

und

$R^{20}$ -die gleiche Bedeutung hat wie $R^{19}$, $R^{18}$ jedoch nicht für Wasserstoff oder eine Schutzgruppe steht,

und

$R^4$ -für Wasserstoff oder

-für Methyl, Ethyl, tert.Butyl, 2,2,2-Trichlorethyl, Allyl, Acetonyl, 4-Nitrobenzyl, 2-Nitrobenzyl, 4-Methoxybenzyl, Benzyl oder Trimethylsilylethyl oder

-für einen Rest der Formel

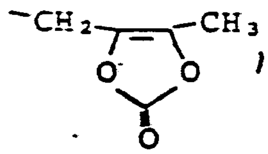 , $-CH(CH_3)OCOOC_2H_5$ oder $-CH_2OCOC(CH_3)_3$

steht.

Die Begriffe Aminoschutzgruppe bzw. Hydroxy-, Mercaptoschutzgruppe haben die bereits vorne angegebene Bedeutung.

Neben den in den Beispielen aufgeführten Produkten sind folgende Verbindungen der allgemeinen Formel (I) besonders bevorzugt:

| $R^1$ | $R^2$ | $R^3$ | $R^4$ |
|---|---|---|---|
| H | 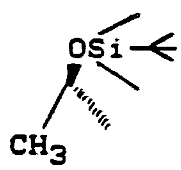 | $PhSO_2$- | $-CH_2-CH=CH_2$ |
| H | " | $CH_3SO_2O$- | " |
| H | " | $CH_3SO_2$- | " |
| H | " | $CH_3S$- | " |
| H | " | $H_2NSO_2$- | " |
| H | " | $H_2NCOS$- | " |

| $R^1$ | $R^2$ | $R^3$ | $R^4$ |
|---|---|---|---|
| H | 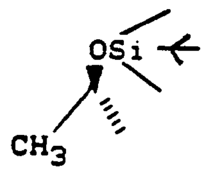 | $(CH_3)_2N$- | $-CH_2-CH=CH_2$ |
| H | " | $H_2N$- | " |
| H | " | $PhCH_2NH$- | " |
| H | " | $CH_3CONH$- | " |
| H | " | CHONH- | " |
| H | " | $CH_3SO_2NH$- | " |
| H | " | $NH_2-C(=NH)-NH-$ | " |
| H | " | $NH_2-C(=O)-NH-$ | " |
| H | " | CHO | " |
| H | " | $CF_3$- | " |
| H | " | $(CH_3O)_2P(=O)-$ | " |

| $R^1$ | $R^2$ | $R^3$ | $R^4$ |
|---|---|---|---|
| H | 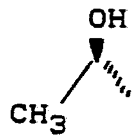 | $COO-CH_2-CH=CH_2$ | $-CH_2-CH=CH_2$ |
| H | " | Ph- | " |
| H | " | Cl- | " |
| H | " | $N_3-$ | " |
| H | " | $CH_3SO_2-$ | " |
| H | " | $CF_3-$ | " |
| H | 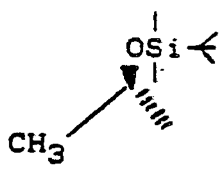 | $PhSO_2-$ | 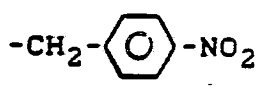 |
| H | " | $CH_3SO_2-$ | " |
| H | " | $CH_3S-$ | " |
| H | " | $H_2NSO_2-$ | " |
| H | " | $H_2NCOS-$ | " |
| H | " | $(CH_3)_2N-$ | " |

| $R^1$ | $R^2$ | $R^3$ | $R^4$ |
|---|---|---|---|
| H | 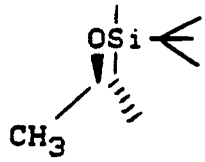 | $H_2N-$ | 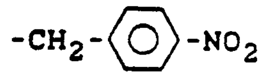 |
| H | " | $PhCH_2NH-$ | " |
| H | " | $CH_3CONH-$ | " |
| H | " | $CHONH-$ | " |
| H | " | $CH_3SO_2NH-$ | " |
| H | " | $H_2N-C(=NH)-NH-$ | " |
| H | " | $H_2N-C(=O)-NH-$ | " |
| H | " | $CHO-$ | " |
| H | " | $CF_3-$ | " |
| H | " | $(CH_3O)_2P(=O)-$ | " |
| H | " | 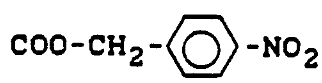 | " |
| H | " | $CH_3SO_2O-$ | " |
| H | " | 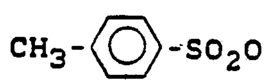 | " |
| 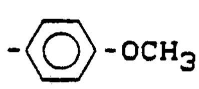 | " | $Cl-$ | $-CH_2-CH=CH_2$ |
| " | " | $N_3-$ | " |

| $R^1$ | $R^2$ | $R^3$ | $R^4$ |
|---|---|---|---|
| 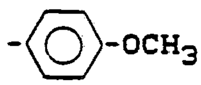 | 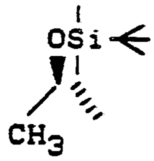 | $CH_3SO_2$ | $-CH_2-CH=CH_2$ |
| " | " | CHONH- | " |
| H | 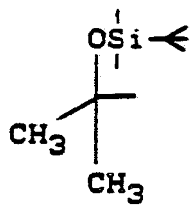 | Cl- | " |
| H | " | $N_3$- | " |
| H | " | $CH_3SO_2$- | " |
| H | " | $PhSO_2$- | " |
| H | " | $CH_3S$- | " |
| H | " | $H_2NSO_2$- | " |
| H | " | $H_2NCOS$- | " |
| H | " | NCS- | " |
| H | " | $(CH_3)_2N$- | " |
| H | " | $H_2N$- | " |
| H | " | $PhCH_2NH$- | " |

| $R^1$ | $R^2$ | $R^3$ | $R^4$ |
| --- | --- | --- | --- |
| H | 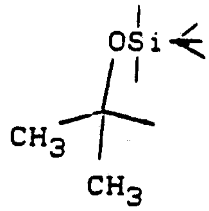 | $CH_3CONH-$ | $-CH_2-CH=CH_2$ |
| H | " | $CH_3SO_2NH-$ | " |
| H | " | $H_2N-C(=NH)-NH-$ | " |
| H | " | $H_2N-C(=O)-NH-$ | " |
| H | " | CHONH- | " |
| H | " | CHO- | " |
| H | " | $CF_3-$ | " |
| H | " | $(EtO)_2P(=O)-$ | " |
| H | " | Cl- | 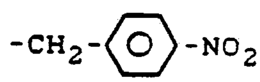 |
| H | " | $N_3-$ | " |
| H | " | $CH_3SO_2-$ | " |
| H | " | $PhSO_2-$ | " |
| H | " | $CH_3S-$ | " |

| $R^1$ | $R^2$ | $R^3$ | $R^4$ |
|---|---|---|---|
| H | 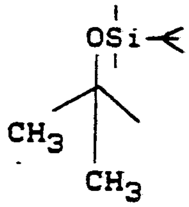 | PhS- | 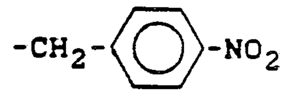 |
| H | " | $H_2NSO_2$- | " |
| H | " | $H_2NCOS$- | " |
| H | " | NCS- | " |
| H | " | $(CH_3)_2N$- | " |
| H | " | $H_2N$- | " |
| H | " | $PhCH_2NH$- | " |
| H | " | $CH_3CONH$- | " |
| H | " | CHONH- | " |
| H | " | $CH_3SO_2NH$- | " |
| H | " | 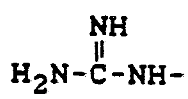 | " |
| H | " | 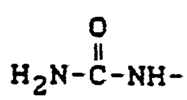 | " |
| H | " | 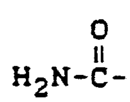 | " |

| $R^1$ | $R^2$ | $R^3$ | $R^4$ |
|---|---|---|---|
| H | 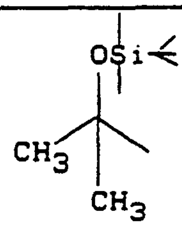 | CHO- | 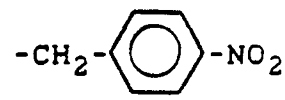 |
| H | " | $CF_3$- | " |
| H | " | 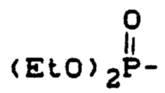 | " |
| H | 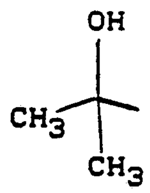 | Cl | " |
| H | " | $CH_3SO_2$- | " |
| H | " | $PhSO_2$- | " |
| H | " | $CH_3S$ | " |

Die Verbindungen der allgemeinen Formel II sind bekannt oder können nach literaturbekannten Methoden hergestellt werden [vgl. Deutsche Offenlegungsschrift 19 06 401; A. Oida et al., Chem. Pharm. Bull 29, 2899 (1981), M. Shiozaki et al., Tetrahedron 39, 2399 (1983); M. Shiozaki et al., Tetrahedron 40 , 1795 (1984), W.J. Leanza et al., Tetrahedron 39, 2505 (1983), T. Kametani et al., J. Chem. Soc. Perkin I 1981, 2228; J.L. Roberts et al., Synthetic Communications 13, 797 (1983)].

Die Verbindungen der allgemeinen Formel III sind bekannt oder können nach literaturbekannten Methoden hergestellt werden [vgl. S. Julia et al., Compt. Rend. Acad. Sci., Paris, Section C 246, 1890 (1967), Europäische Patentanmeldungsschrift 78 026, EP 52 299, R.M. Kellogg et al., J.C.S. Chem. Comm. (1977)932].

Als Verdünnungsmittel kommen alle inerten Lösungsmittel in Frage. Dazu gehören vorzugsweise Ether wie Dimethoxyethan, Diglyme, Triglyme, Tetrahydrofuran, Dioxan, Diethylether oder tert.Butylmethylether, halogenierte Kohlenwasserstoffe wie Dichlormethan, Trichlormethan, Tetrachlormethan, 1,1,2-Trichlorethan, Dichlorethan oder Trichlorethylen, Chlorbenzyl, Dichlorbenzol, Essigsäureethylester. Toluol oder Cyclohexan.

Die Umsetzung erfolgt im allgemeinen bei Temperaturen von -30°C bis +50°C, vorzugsweise bei Raumtemperatur. Als Basen eignen sich alle tertiären Amine, Bevorzugt zu nennen sind Triethyl-, Tripropyl-oder Tributylamin, Ethyldiisopropylamin, Pyridin, Picolin, Lutidin, N-Methylmorpholin, N-Methylpiperidin, 1,5-Diazabicyclo [5,4-0]undec-5-en - (DBU) oder 1,5-Diazabicyclo[4,3,0]-non-3-en (DBN).

Als Silylierungsmittel bzw. Katalysatoren kommen alle Silylierungsmittel in Betracht, die gleichzeitig als Lewis-Säuren wirken können. Hierzu gehören vorzugsweise Trialkylsilylperfluoralkansulfonate, Trialkylsilylalkansulfonate, Trialkylsilylarylsulfonate, Trialkylsilylper fluoralkanoate, Trialkylsilylalkanoate, Trialkylsilylhalogenide, Trialkylsilylperchlorate, Trialkylsilyldifluorphosphate, Trialkylsilyldichlorphosphate, Trialkylsilyfluorosulfonate, N,O-Bis(trialkylsilyl)-acetamide, Trialkylsilylcyanide. Besonders geeignet sind Trimethylsilyltrifluormethansulfonat, Trimethylsilylnonafluorobutansulfonat, Trimethylsilyltrifluoracetat, Trimethylsilylperchlorat, Trimethylsilylbromid, Trimethylsilyldifluorphosphat, Trimethylsilylfluorsulfonat, N,O-Bis(trimethylsilyl)trifluoracetamid.

Für die Durchführung des Verfahrens ist es notwendig zunächst

a) pro Äquivalent der Verbindung III 0,1-1. bevorzugt 0,5-1 Äquivalent der Verbindung II,

b) pro Äquivalent der Verbindung III 1-2. bevorzugt 1-1,5 Äquivalent Silylierungsmittel,

c) pro Äquivalent Silylierungsmittel. 1,01-3. bevorzugt 1,05-2 Äquivalent Base (Amin)

einzusetzen und nach 0,1-24 h, bevorzugt 0,1-12 h soviel weiteres Silylierungsmittel zuzugeben; daß dann das Silylierungsmittel in einem geringen Überschuß, bevorzugt in einem katalytischen Überschuß über die Base (Amin) vorhanden ist.

Bei der Durchführung des Verfahrens sind außer den oben angegebenen Mengenverhältnissen an Silylierungsmittel und Base (Amin) für jede freie, d.h. zu schützende Hydroxy-, Mercapto-bzw. Amingruppe in den Verbindungen II und III jeweils ein zusätzliches Äquivalent Silylierungsmittel und Base (Amin) enzusetzen (z.B. wenn R' = H).

Die Umsetzungsgeschwindigkeit ist weitgehend von der Menge des verwendeten Katalysators d.h. des Silylierungsmittelüberschusses abhängig. Die Umsetzung erfolgt innerhalb 0,1 bis 48 h, vorzugsweise von 0,1 bis 24 h.

Die Verbindungen der allgemeinen Formel I sind wertvolle Zwischenprodukte für die Synthese wirksamer Antibiotika [vgl. u.a. D.H. Shih et al., Heterocycles 21, 29 (1984)].

Die folgenden Beispiele dienen der weiteren Beschreibung der Erfindung, ohne diese zu beschränken.

A) Beispiele zur Herstellung von Verbindungen der Formel III:

Herstellungsbeispiel 1

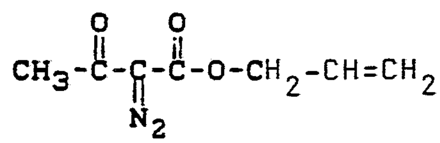

Allyl-2-diazo-3-oxo-butanoat

Eine auf 0°C gekühlte Lösung von 28,43 g (0,2 mol) Acetessigsäureallylester und 43,39 g (0,22 mol -1.1 equiv.) p-Toluolsulfonylazid in 400 ml wasserfreiem Acetonitril wurde tropfenweise mit 55,4 ml (0,4 mol -2 equiv.) Triethylamin versetzt. Die Mischung wurde 3,5 h bei 0°C gerührt, mit 50 g Kieselgur versetzt, im Vakuum eingedampft und an 400 g Kieselgel (Toluol:Ethylacetat 96:4) chromatographiert. Man erhielt 31,9 g (95 %) der Titelverbindung als hellgelbes Öl, Rf: 0,36 (Toluol:Ethylacetat 9:1).

IR ($CHCl_3$) 2141 ($N_2$), 1714 (C=O) , 1651 $cm^{-1}$ - (C=O).

$^1$H-NMR (200 MHz, $CDCl_3$) δ 2.50 (s, 3H, $CH_3$), 4.76 (m, 2H, -$\underline{CH_2}$-CH=$CH_2$), 5,3-5,4 (m, 2H, -$CH_2$-CH=$\underline{CH_2}$), 5,9-6,1 (m, 1H, -$CH_2$-$\underline{CH}$-=$CH_2$).

$C_7H_8N_2O_3$ (168,2) Ber.: C 50,00 H 4,80 N 16,66

Gef.: C 49,9 H 4,8 N 16,6

Herstellungsbeispiel 2

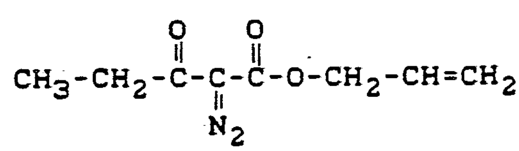

Allyl-2-diazo-3-oxo-pentanoat

a.) In Analogie zu der von L. Weiler et.al., J.Am.Chem. Soc. 96, 1082 (1974) beschriebenen Methode erhielt man aus 28,43 g (0,2 mol) Allyl-3-oxo-butanoat und 15 ml (0,24 mol -1,2 equiv.) Methyljodid und Chromatographie des Rohprodukts an 700 g Kieselgel (Toluol:Ethylacetat 95:5) und anschließende Destillation 8,3 g (27 %) Allyl-3-oxo-pentanoat als farblose Flüssigkeit, Siedepunkt 84°C/14 mm Hg, Rf: 0,34 (Toluol: Ethylacetat 9:1).

IR ($CHCl_3$) 1741 (C=O), 1714 $cm^{-1}$ (C=O)

$^1$H-NMR (300 MHz, $CDCl_3$) δ 1,10 (t, J=8 Hz, 3H, $CH_2\underline{CH_3}$), 2,60 (q, J=8 Hz, 2H, $\underline{CH_2}CH_3$), 3,48 (s, 2H, $CH_2$), 4,65 (m, 2H, $\underline{CH_2}$-CH=$CH_2$), 5,2-5,4 (m, 2H, CH=$\underline{CH_2}$), 5,8-6,0 (m, 1H, $CH_2$-$\underline{CH}$=$CH_2$).

$C_8H_{12}O_3$ (156,2) Ber.: C 61,52 H 7,74

Gef.: C 61,4 H 7,8

b.) Wie für Herstellungsverfahren 1 beschrieben erhielt man aus 8,3 g (53,1 mmol) Allyl-3-oxo-pentanoat und Chromatographie des Rohproduktes an 130 g Kieselgel - (Toluol:Ethylacetat 96:4) 9,34 g (97 %) der Titelverbindung als hellgelbes Öl, Rf: 0,5 (Toluol:Ethylacetat 9:1)

IR ($CHCl_3$) 2137 ($N_2$), 1712 (C=O), 1650 $cm^{-1}$ (C=O).

$^1$H-NMR (200 MHz, $CDCl_3$) δ 1,16 (t, J=8 Hz, 3H, $CH_3$), 2,89 (q, J=8 Hz, 2H, $CH_2CH_3$), 5,52 (m, 2H, $CH_2$-CH=CH), 5,3-5,4 (m, 2H, CH= $CH_2$), 5,9-6,1 (m, 1H, $CH_2$-CH=$CH_2$).

$C_8H_{10}N_2O_3$ (182,2) Ber.: C 52,74 H 5,53 N 15,38
Gef.: C 52,1 H 5,5 N 14,8

Herstellungsbeispiel 3

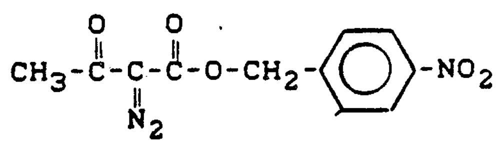

4-Nitrobenzyl-2-diazo-3-oxo-butanoat

Eine auf 0°C gekühlte Lösung von 33,18g (0,14 mol) Acetessigsäure-4-nitrobenzylester und 30,40 g (0,15 mol) p-Toluolsulfonylazid in 280 ml wasserfreiem Acetonitril wurde tropfenweise mit 35,0 ml (0,25 mol -1,8 equiv.) Triethylamin versetzt. Die Mischung wurde 2 h bei 0°C gerührt, dann wurde der entstandene Niederschlag abgesaugt, mit Ether gewaschen und über $P_4O_{10}$ im Hochvakuum getrocknet. Man erhielt 20,3 g (55 %) der Titelverbindung als farblose Kristalle, Schmelzpunkt 132°C. Durch Chromatographie der Filtratlösung an 400 g Kieselgel - (Toluol:Ethylacetat 9:1) wurden weitere 6,3 g (17 %) der Titelverbindung erhalten, Rf: 0,35 (Toluol:Ethylacetat 9:1).

IR (KBr) 2144 ($N_2$), 1709 (C=O), 1662 (C=O), 1514 ($NO_2$ asym.), 1343 $cm^{-1}$ ($NO_2$ sym.).

$^1$H-NMR (200 MHz, DMSO) δ 2,44 (s, 3H, $CH_3$), 5,44 (s, 2 H, $CH_2$), 7,72 (d, J=9 Hz, 2H, $H_{arom.}$), 8,28 (d, J=9 Hz, 2H, $H_{arom.}$).

$C_{11}H_9N_3O_5$ (263,2) Ber.: C 50,20 H 3,45 N 15,96
Gef.: C 50,2 H 3,4 N 15,9

Herstellungsbeispiel 4

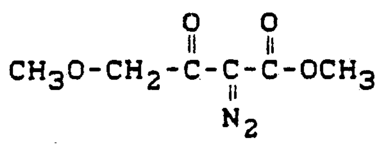

Methyl-2-diazo-4-methoxy-3-oxo-butanoat

Wie für Herstellungsbeispiel 1 beschrieben erhielt man aus 2,92 g (20 mmol) 4-Methoxyacetessigsäuremethylester und Chromatographie des Rohproduktes an 900 g Kieselgel (Toluol:Ethylacetat 3:1) 3,05 g (89 %) der Titelverbindung als hellgelbes Öl, Rf: 0,35 (Toluol:Ethylacetat 3:1).

IR ($CHCl_3$) 2138 ($N_2$), 1711 (C=O), 1665 $cm^{-1}$ - (C=O).

$^1$H-NMR 250 MHz, $CDCl_3$) δ 3,48 (s, 3H, $COOCH_3$), 3,86 (s, 3H, $OCH_3$), 4,53 (s, 2H, $CH_2$).

| | | | | |
|---|---|---|---|---|
| $C_6H_8N_2O_4$ (172,1) | Ber.: | C 41,86 | H 4,68 | N 16,27 |
| | Gef.: | C 42,1 | H 4,7 | N 16,6 |

Herstellungsbeispiel 5

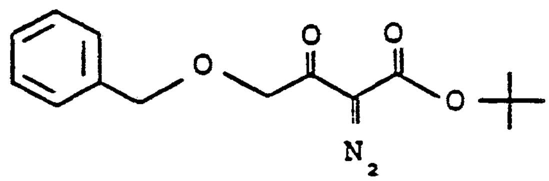

tert.-Butyl-4-benzyloxy-2-diazo-3-oxo-butanoat

Eine auf 0°C gekühlte Lösung von 5,29 g (20 mmol) 4-Benzyloxyacetessigsäure-tert.-butylester und 4,34 g (22 mmol) p-Toluolsulfonsäureazid in 40 ml wasserfreiem Acetonitril wurde tropfenweise mit 5,54 ml (40 mmol -2 equiv.) Triethylamin versetzt. Die Mischung wurde 30 min. bei 0°C gerührt, mit 8 g Kieselgur versetzt, im Vakuum eingedampft und an 100 g Kieselgel (Toluol:Ethylacetat 95:5) chromatographiert. Man erhielt 3,98 g (69 %) der Titelverbindung als farblose Kristalle, Schmelzpunkt: 68°C, Rf: 0,31 - (Toluol:Ethylacetat 95:5).

IR (KBr) 2144 $N_2$), 1700, 1673 $cm^{-1}$.

$^1$H-NMR (300 MHz, $CDCl_3$) δ 1,51 (s, 9H, $C(CH_3)_3$), 4,59 (s, 2H, Ch₂), 4,67 (s, 2H, $CH_2$), 7,3-7,5 (m, 5H, Ph).

| | | | | |
|---|---|---|---|---|
| $C_{15}H_{18}N_2O_4$ (290,3) | Ber.: | C 62,06 | H 6,23 | N 9,65 |
| | Gef.: | C 62,1 | H 6,2 | N 9,6 |

Herstellungsbeispiel 6

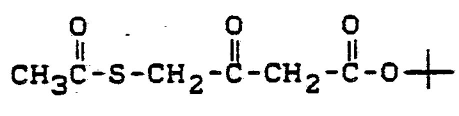

4-Acetylthioacetessigsäure-tert.-butylester

Zu einer auf 0°C gekühlte Lösung von 3,85 g (20,0 mmol) 4-Chloracetessigsäure-tert.-butylester in 40 ml wasserfreiem Acetonitril gab man 2,40 g (21,0 mmol) trockenes Kaliumthioacetat und rührte 30 min. bei dieser Temperatur. Danach goß man die Mischung in ein Gemisch aus NaCl-Lösung und Ethylacetat, trennte die organische Phase ab. extrahierte mit 2 x 30 ml Ethylacetat, wusch mit Wasser und trocknete über $MgSO_4$. Nach Abdampfen des Lösungsmittels im Vakuum und Chromatographie des Rückstandes an 60 g Kieselgel (Toluol:Ethylacetat 95:5) erhielt man 3,87 g (83 %) der Titelverbindung als farbloses Öl, Rf: 0,24 (Toluol:Ethylacetat 95:5).

IR ($CHCl_3$) 1740-1710 $cm^{-1}$ (C=O).

$^1$H-NMR (250 MHz, $CDCl_3$) δ 1,50 (s, 9H, $C(CH_3)_3$), 2,42 (s, 3H, $CH_3CO$), 3,53 (s, 2H, $CH_2$), 3,90 (s, 2H, $CH_2$).

Herstellungsbeispiel 7

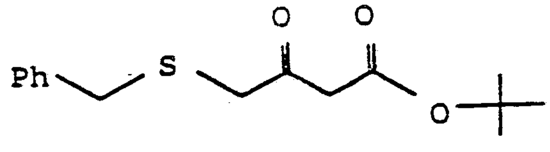

4-Benzylthioacetessigsäure-tert.-butylester

Zu einer Suspension von 1,8 g (60 mmol) Natriumhydrid (80 % in Paraffinöl) in 50 ml wasserfreiem THF wurden bei Raumtemperatur langsam 7,04 (60 mmol) Benzylmercaptan getropft. Dann wurde 15 min. nachgerührt und anschließend auf 0°C abgekühlt. Bei dieser Temperatur gab man innerhalb von 1 h eine Lösung von 10,57 g - (55 mmol) 4-Chloracetessigsäure-tert.-butylester in 23 ml THF zu. Das Eisbad wurde entfernt, man rührte noch 1 h bei Raumtemperatur und neutralisierte die Mischung durch Zugabe einigr Tropfen 10 %iger HCl. Das Lösungsmittel wurde im Vakuum abgedampft, man versetzte mehrmals mit Ether, wusch die Extrakte mit Wasser und trocknete über $MgSO_4$. Abdampfen des Ethers im Vakuum ergab ein Öl, welches durch Chromatographie an 800 g Kieselgel (Toluol) gereinigt wurde. Man erhielt 12,74 g (83 %) der Titelverbindung in Form eines farblosen Öls, Rf: 0,13 - (Toluol).

IR ($CHCl_3$) 1740-1710 $cm^{-1}$ (C=O, β-Ketoester).

$^1$H-NMR (200 MHz, $CDCl_3$) δ 1,49 (s, 9H, $C(CH_3)_3$), 3,22 (s, 2H, $CH_2$), 3,52 (s, 2H, $CH_2$), 3,68 (s, 2H, $CH_2$), 7,33 (s, 5H, Ph).

| | | | | | |
|---|---|---|---|---|---|
| $C_{15}H_{20}O_3S$ (280,4) | Ber.: | C 64,3 | H 7,2 | S 11,4 |
| | Gef.: | C 64,3 | H 7,2 | S 11,3 |

Herstellungsbeispiel 8

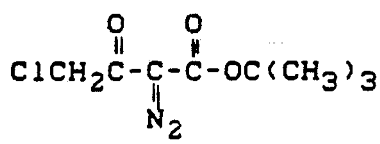

tert.-Butyl-4-chloro-2-diazo-3-oxo-butanoat

Wie für Herstellungsbeispiel 1 beschrieben erhielt man aus 2,79 g (14,5 mmol) 4-Chloracetessigsäure-tert.-butylester und Filtration des Rohprodukts an 30 g Kieselgel - (Toluol) 2,67 g (84 %) der Titelverbindung als Kristalle, Rf: 0,29 (Toluol) Schmelzpunkt: 40-41°C.

IR ($CHCl_3$); 2140 ($N_2$), 1708 (C=O), 1662 $cm^{-1}$.

$^1$H-NMR (300 MHz, $CDCl_3$) δ 1,54 (s, 9H, $C(CH_3)_3$), 4,60 (s, 2H, $CH_2$).

| | | | | | |
|---|---|---|---|---|---|
| $C_8H_{11}N_2O_3Cl$ (218,6) | Ber.: | C 43,9 | H 5,1 | N 12,8 |
| | Gef.: | C 44,0 | H 5,1 | N 13,3 |

Herstellungsbeispiel 9

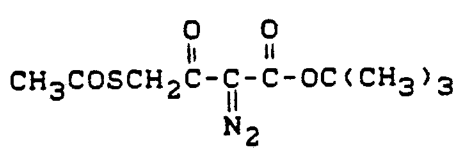

tert.-Butyl-4-acetylthio-2-diazo-3-oxo-butanoat

Wie für Herstellungsbeispiel 1 beschrieben erhielt man aus 6,97 g (30,0 mmol) 4-Acetylthioacetessigsäure-tert.-butylester nach Chromatographie des Rohprodukts an 280 g Kieselgel 3,48 g (45 %) der Titelverbindung als Öl, Rf-0,34 (Toluol:Ethylacetat 95:5).

IR ($CHCl_3$) 2140 ($N_2$), 1705 (C=O), 1656 $cm^{-1}$.

$^1$H-NMR (300 MHz, $CDCl_3$), 1,55 (s, 9H, $C(CH_3)_3$), 2,39 (s, 3H, $COCH_3$), 4,23 (s, 2H, $CH_2$).

| | | | | |
|---|---|---|---|---|
| $C_{10}H_{14}N_2O_4S$ (258,3) | Ber.: | C 46,5 | H 5,5 | N 10,8 |
| | Gef.: | C 46,5 | H 5,4 | N 11,3 |

Herstellungsbeispiel 10

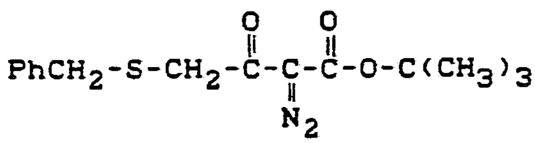

tert.Butyl-4-benzylthio-2-diazo-3-oxo-butanoat

Wie für Herstellungsbeispiel 1 beschrieben erhielt man aus 4,21 g (15,0 mmol) 4-Benzylthioacetessigsäure-tert. butylester nach Chromatographie des Rohprodukts an 100 g Kieselgel (Toluol) 3,4 g (74 %) der Titelverbindung als gelbes Öl, Rf: 0,28 (Toluol).

IR ($CHCl_3$) 2145 ($N_2$), 1705 1635 $cm^{-1}$
$^1$H-NMR (250 MHz, $CDCl_3$) $\delta$ 1,52 (s, 9H, $C(CH_3)_3$), 3,58 (s, 2H, $CH_2$), 3,77 (s, 2H, $CH_2$), 7,3-7,4 (m, 5H, Ph).

| | | | | |
|---|---|---|---|---|
| $C_{15}H_{18}N_2O_3S$ (306,38) | Ber.: | C 58,80 | H 5,92 | S 10,46 |
| | Gef.: | C 59,2 | H 6,0 | S 10,5 |

Herstellungsbeispiel 11

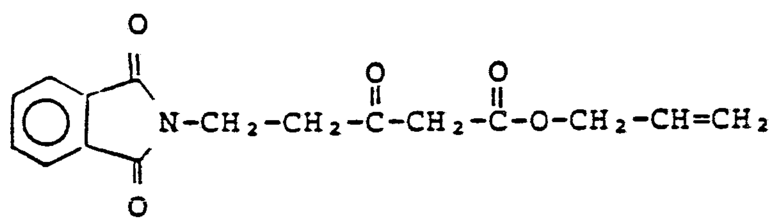

Allyl-3-oxo-5-pthalimido-pentanoat

Eine Lösung von 2,89 g (10,0 mmol) Ethyl-3-oxo-5-pthalimidopentanoat in 14 ml Allylalkohol wurde in Gegenwart katalytischer Mengen an p-Toluol-sulfonsäurehydrat 3 Tage auf 80°C erwärmt. Danach wurd das Lösungsmittel im Vakuum abgedampft und der Rückstand durch Chromatographie an 170 g Kieselgel (Toluol:Ethylacetat 4:1) gereinigt. Man erhielt die Titelverbindung als farblose Kristalle, Schmelzpunkt; 53-55°C, Rf: 0,40 (Toluol.Ethylacetat 4:1).

IR ($CHCl_3$) 1716 $cm^{-1}$ (C=O).

$^1$H-NMR (300 MHz, $CDCl_3$) $\delta$ 3,01 (t, J=7 Hz, 2H, $CH_2CO$), 3,54 (s, 2H, $COCH_2CO$), 4,0 (t, J=7 Hz, 2H, $CH_2N$), 4,65 (m, 2H, $\underline{CH_2}$-CH=$CH_2$), 5,2-5,4 (m, 2H, CH=$\underline{CH_2}$), 5,8-6,0 (m, 1H, $CH_2$-$\underline{CH}$=$CH_2$), 7,73, 7,87 (m, je 2 H, $H_{arom.}$).

$C_{16}H_{15}NO_5$ (301,3) Ber.: C 63,78 H 5,02 N 4,64
Gef.: C 63,8 H 5,4 N 4,3

Herstellungsbeispiel 12

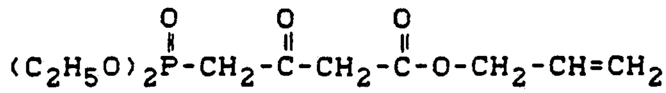

Allyl-4-(diethylphosphinyl)-3-oxo-butanoat

Zu einer auf -30°C gekühlten Suspension von 300 mg (10,0 mmol) Natriumhydrid in THF tropfte man langsam eine Lösung von Allyl-4-brom-3-oxo-butanoat (Deutsche Offenlegungsschrift 2 048 470) in 10 ml THF. Es wurde 15 min. bei -30°C gerührt, dann tropfte man 0,65 ml (5,02 mmol) Diethylphosphit zu. Das Kühlbad wurde entfernt und man rührte 30 min. bei Raumtemperatur nach. Zur Aufarbeitung goß man in ein Gemisch aus Ethylacetat und gesättigter $NaHCO_3$-Lösung, extrahierte mit Ethylacetat, wusch mit gesättigter NaCl-Lösung und trocknete über $MgSO_4$.

Nach Abdampfen des Lösungsmittels im Vakuum und Chromatographie des Rückstandes an 90 g Kieselgel - (Toluol:Ethylacetat 1:4) erhielt man 705 mg (51 %) der Titelverbindung als Öl, Rf: 0,30 (Toluol:Ethylacetat 1:4).

IR ($CHCl_3$) 1735, 1718, 1640, 1250 $cm^{-1}$ (P=O).

$^1$H-NMR 300 MHz, $CDCl_3$) $\delta$ 1,35 (t, J=7,5 Hz, 6H, $\underline{CH_3}CH_2$), 3,26 (d, J=23 Hz, 2H, $PCH_2CO$), 3,71 (s, 2H, $COCH_2CO$), 4,17 (q, J=7,5 Hz, 4H, $CH_3\underline{CH_2}$), 4,66 (m, $\underline{CH_2}$-CH=$CH_2$), 5,2-5,4 (m, 2H, CH=$\underline{CH_2}$), 5,8-6,0 (m, 1H, $CH_2$-$\underline{CH}$=$CH_2$).

Herstellungsbeispiel 13

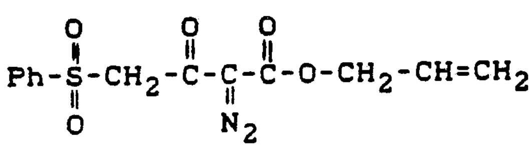

Allyl-2-diazo-3-oxo-4-phenylsulfonyl-butanoat

Eine auf 0°C gekühlte Lösung von 1.38 g (5.0 mmol) Allyl-2-diazo-3-oxo-4-phenylthio-butanoat in 10 ml Dichlormethan wurde mit 2.59 g(15.0 mmol) m-Chlorperbenzoesäure versetzt und 1 h bei dieser Temperatur gerührt. Zur Aufarbeitung wurde in gesättigte $NaHCO_3$-Lösung geschüttet, mit Ethylacetat extrahiert, mit Wasser gewaschen und über $MgSO_4$ getrocknet. Nach Abdampfen des Lösungsmittels i.Vak. und Filtration des Rohprodukts and 30 g Kieselgel (Toluol:Ethylacetat 9:1) erhielt man 883 mg (57 %) der Titelverbindung, Rf: 0.24 (Toluol:Ethylacetat 95:5).

IR ($CHCl_3$) 2150 ($N_2$), 1716, 1647, 1328 ($SO_2$ as.), 1160 $cm^{-1}$ ($SO_2$ sym.).

$^1$H-NMR (200 MHz, $CDCl_3$) $\delta$ 4.78 (d, J=7 Hz, 2H, $\underline{CH_2}$-CH=$CH_2$), 4.88 (s, 2H, $SO_2CH_2CO$), 5.4-5.5 (m, 2H, CH=$\underline{CH_2}$), 5.9-6.1 (m, 1H, $CH_2$-$\underline{CH_2}$=$CH_2$), 7.55-7.75 (m, 3H, Ph), 8.02 (m, 2H, Ph).

$C_{13}H_{12}N_2O_5S$ (308.3) Ber.: C 50.64 H 3.92 N 9.09 S 10.40
Gef.: C 50.3 H 3.9 N 8.9 S 10.4

Herstellungsbeispiel 14

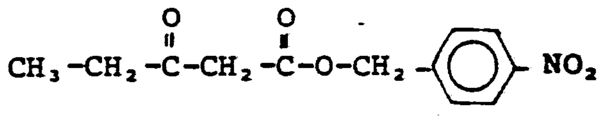

4-Nitrobenzyl-3-oxo-pentanoat

Eine Suspension von 55.3 g (0.36 mol) 4-Nitrobenzylalkohol und 56.2 g (0.39 mol) Ethyl-3-oxopentanoat in 360 ml Toluol wurde zum Sieden erhitzt.

Innerhalb von 10 h wurden ca. 300 ml Toluol abdestilliert dann wurde abgekühlt, man gab nochmals 200 ml zu und wiederholte diesen Vorgang. Nach dem Abkühlen wurde durch Kieselgur filtriert, mit Toluol nachgewaschen und die Filtratlösung i.Vak. eingedampft. Man erhielt 89 g - (98%) der Titelverbindung als Öl, Rf: 0.42 - (Toluol:Ethylacetat 4:1).

IR ($CHCl_3$), 1751 (C=O), 1722 (C=O), 1524 ($NO_2$ asym.), 1351 $cm^{-1}$ ($NO_2$ sym.).

$^1$H-NMR (300 MHz, $CDCl_3$) $\delta$ 1.10 (t, J=7.5 Hz, 3H, $\underline{CH_3}CH_2$), 2.59 (q, J=7.5 Hz, 2H, $CH_3\underline{CH_2}$), 3.57 (s, 2H, $COCH_2CO$), 5.29 (s, 2H, $CO_2CH_2$), 7.53 (d, J=9 Hz, 2H, $H_{arom.}$), 8.20 (d, J=9 Hz, 2H, $H_{arom.}$).

Analog den bereits beschriebenen Herstellungsbeispielen wurden außerdem folgende 4-substituierte Acetessigester erhalten (Herstellungsbeispiele 15 -21, Tabelle 1):

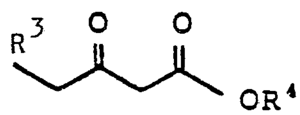

Aus diesen erhielt man wie für Herstellungsbeispiel 1 beschrieben durch Diazotransfer die entsprechenden Diazoverbindungen (Herstellungsbeispiele 22 -34, Tabelle 2):

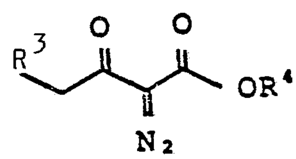

Tabelle 1:

| Herst. beisp. No | $R^3$ | $R^4$ | hergestellt nach Herst.beisp. | Ausb. % | Rf (Tol.:Ethylac.) | IR ($cm^{-1}$) | 1-HNMR (200 MHz, $CDCl_3$) δ ppm | Analyse |
|---|---|---|---|---|---|---|---|---|
| 15 | $CH_3COS-$ | $-CH_2-CH=CH_2$ | 6 | 73 | 0.24 (95:5) | 1724<br>1697 | 2.36 (s, 3H, $CH_3CO$),<br>3.60 (s, 2H, $COCH_2CO$),<br>3.84 (s, 2H, $\underline{CH_2}SCOCH_3$),<br>4.62 (m, 2H, $\underline{CH_2}-CH=CH_2$),<br>5.2-5.4 (m, 2H, $CH=\underline{CH_2}$),<br>5.8-6.0 (m, 1H, $CH_2-\underline{CH}=CH_2$), | $C_9H_{12}O_4S$ (216.3)<br>Ber.: C 49.99 H 5.59 S 14.82<br>Gef.: C 50.0 H 5.6 S 14.7 |
| 16 | 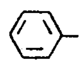 | $-CH_2-CH=CH_2$ | 11 | 40 | 0.36 (95:5) | 1744<br>1721 | 3.50 (s, 2H, $COCH_2CO$),<br>3.85 (s, 2H, $\underline{CH_2}Ph$),<br>4.63 (m, 2H, $\underline{CH_2}-CH=CH_2$),<br>5.2-5.4 (m, 2H, $CH=\underline{CH_2}$),<br>5.8-6.0 (m, 1H, $CH_2-\underline{CH}=CH_2$). | $C_{13}H_{14}O_3$ (218.2)<br>Ber.: C 71.54 H 6.47<br>Gef.: C 71.5 H 6.7 |
| 17 | 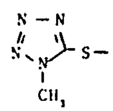 | $-CH_2-CH=CH_2$ | 6 | 86 | 0.27 (7:3) | 1738 (sh)<br>1719<br>1650 | 3.74 (s, 2H, $COCH_2CO$),<br>3.97 (s, 3H, $NCH_3$),<br>4.41 (s, 2H, $SCH_2CO$),<br>4.76 (m, 2H, $\underline{CH_2}-CH=CH_2$),<br>5.25-5.4 (m, 2H, $CH=\underline{CH_2}$),<br>5.8-6.0 (m, 1H, $CH_2-\underline{CH}=CH_2$). | $C_9H_{12}N_4O_3S$ (256.3)<br>Ber.: C 42.18 H 4.72 N 21.86 S 12.51<br>Gef.: C 42.0 H 4.7 N 21.5 H 12.5 |
| 18 | $N_3$ | $-CH_2-CH=CH_2$ | 12, jedoch nur 1 equiv. NaH. | 43 | 0.34 (95:5) | 2110 ($N_3$)<br>1740 (sh)<br>1725 | 3.58 (s, 2H, $COCH_2CO$),<br>4.15 (s, 2H, $N_3CH_2CO$),<br>4.69 (m, 2H, $\underline{CH_2}-CH=CH_2$),<br>5.3-5.5 (m, 2H, $CH=\underline{CH_2}$),<br>5.9-6.1 (m, 1H, $CH_2-\underline{CH}=CH_2$). | $C_7H_9N_3O_3$ (183.2)<br>Ber.: C 45.90 H 4.95<br>Gef.: C 45.8 H 4.9 |
| 19 | $COO-CH_2-CH=CH_2$ | $-CH_2-CH=CH_2$ | 11 | 28 | 0.29 (96:4) | 1735<br>1720<br>1655 | 3.66 (s, 4H, $COCH_2CO$),<br>4.64 (m, 4H, $\underline{CH_2}-CH=CH_2$),<br>5.25-5.3 (m, 4H, $CH=\underline{CH_2}$),<br>5.85-6.0 (m, 2H, $CH_2-\underline{CH}=CH_2$). | $C_{11}H_{14}O_5$ (226.2)<br>Ber.: C 58.40 H 6.24<br>Gef.: C 58.4 H 6.3 |
| | | | aus Dimethylester | + | | | | |
| | $COOCH_3$ | $-CH_2-CH=CH_2$ | | 16 | 0.21 (96:4) | 1740<br>1720<br>1650 | 3.64 (s, 2H, $CH_2$),<br>3.66 (s, 2H, $CH_2$),<br>3.77 (s, 3H, $COOCH_3$),<br>4.66 (m, 2H, $\underline{CH_2}-CH=CH_2$),<br>5.25-5.4 (m, 2H, $CH=\underline{CH_2}$),<br>5.8-6.0 (m, 1H, $CH_2-\underline{CH}=CH_2$). | $C_9H_{11}O_5$<br>Ber.: C 54.27 H 5.57<br>Gef.: C 54.0 H 6.0 |

Fortsetzung Tabelle 1

| Herst. beisp. No. | $R^3$ | $R^4$ | hergestellt nach Herst.beisp. | Ausb. % | Rf (Tol.:Ethylac.) | IR ($cm^{-1}$) | $^1$H-NMR (200 MHz, $CDCl_3$) $\delta$ ppm | Analyse |
|---|---|---|---|---|---|---|---|---|
| 20 | 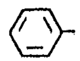 | 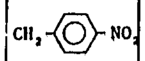 | 14 | 98 | 0.31 (95:5) | - | 3.54 (s, 2H, $COCH_2CO$), 3.86 (s, 2H, $CH_2Ph$), 5.27 (s, 2H, $COOCH_2$), 7.51 (d, J=9 Hz, 2H, Harom.), 8.20 (d, J=9 Hz, 2H, Harom.). | - |
| 21 | 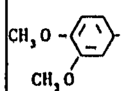 | 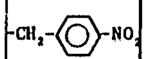 | 14 | 97 | 0.40 (7:3) | - | 3.55 (s, 2H, $CH_2$), 3.76 (s, 2H, $CH_2$), 3.85 (s, 3H, $OCH_3$), 3.86 (s, 3H, $OCH_3$), 5.23 (s, 2H, $CO_2CH_2$), 6.7-6.9 (m, 3H, Harom.), 7.50 (d, J=9 Hz, 2H, Harom.), 8.22 (d, J=9 Hz, 2H, Harom.). | - |
| 21 (a) | $CF_3$ | $-CH_2-CH=CH_2$ | acc. to K.P. Zeiler Synthesis (1985) 334. | 22 | 0.42 (9:1) | 1718 1650 | 3.46 (q, J=11 Hz, 2H, $CF_3\underline{CH_2}$) 3.60 (s, 2H, $COCH_2CO$), 4.67 (m, 2H, $-CH_2-CH=CH_2$), 5.2-5.4 (m, 2H, $-CH=\underline{CH_2}$), 5.85-6.0 (m, 1H, $-\underline{CH}=CH_2$). | bp.: 140-150°C/ 10 mm (Kugelrohr) |

Tabelle 2:

| Herst. beisp. No | $R^3$ | $R^4$ | Ausb. % | Rf (Tol.:Etyhlac.) | IR ($cm^{-1}$) | $^1H$-NMR (200 MHz, $CDCl_3$) $\delta$ ppm | Analyse |
|---|---|---|---|---|---|---|---|
| 22 | Cl | $-CH_2-CH=CH_2$ | 76 | 0.24 (1:0) | 2147 ($N_2$)<br>1714<br>1666 | 4.64 (s, 2H, $CH_2Cl$),<br>4.76 (m, 2H, $CH_2-CH=CH_2$),<br>5.3-5.4 (m, 2H, $CH=CH_2$),<br>5.85-6.05 (m, 1H, $CH_2-CH=CH_2$). | $C_7H_7ClN_2O_3$ (202.6)<br>Ber.: C 41.50 H 3.48 N 13.8<br>Gef.: C 41.7 H 3.6 N 13.8 |
| 23 | $CH_3COS$ | $-CH_2-CH=CH_2$ | 89 | 0.31 (95:5) | 2144 ($N_2$)<br>1715<br>1658<br>1327 | 2.39 (s, 3H, $CH_3CO$),<br>4.25 (s, 2H, $CH_2SCOCH_3$),<br>4.77 (m, 2H, $CH_2-CH=CH_2$),<br>5.3-5.4 (m, 2H, $CH=CH_2$),<br>5.9-6.0 (m, 1H, $CH_2-CH=CH_2$). | $C_9H_{10}N_2O_4S$ (242.3)<br>Ber.: C 44.62 H 4.16 N 11.56 S 13.23<br>Gef.: C 45.0 H 4.2 N 11.5 H 13.6 |
| 24 | 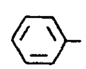 | $-CH_2-CH=CH_2$ | 88 | 0.21 (1:0) | 2143 ($N_2$)<br>1715<br>1649 | 4.21 (s, 2H, $PhCH_2$),<br>4.77 (m, 2H, $CH_2-CH=CH_2$),<br>5.3-5.4 (m, 2H, $CH=CH_2$),<br>5.9-6.1 (m, 1H, $CH_2-CH=CH_2$),<br>7.32 (m, 5H, Ph). | $C_{13}H_{12}N_2O_3$ (244.3)<br>Ber.: C 63.93 H 4.95 N 11.47<br>Gef.: C 63.8 H 5.0 N 11.7 |
| 25 | 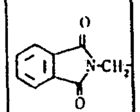 | $-CH_2-CH=CH_2$ | 80 | 0.34 (9:1) | 2143 ($N_2$)<br>1718<br>1650 | 3.25 (t, J=7 Hz, 2H, $CH_2CO$),<br>4.05 (t, J=7 Hz, 2H, $CH_2N$),<br>4.70 (m, 2H, $CH_2-CH=CH_2$),<br>5.3-5.4 (m, 2H, $CH=CH_2$),<br>5.8-6.0 (m, 1H, $CH_2-CH=CH_2$),<br>7.70, 7.85 (m, je 2H, Harom). | $C_{16}H_{13}N_3O_5$ (327.3)<br>Ber.: C 58.72 H 4.00 N 12.84<br>Gef.: C 58.7 H 4.0 N 12.8 |
| 26 | 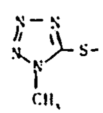 | $-CH_2-CH=CH_2$ | 97 | 0.23 (4:1) | 2134 ($N_2$)<br>1712<br>1655 | 4.01 (s, 3H, $NCH_3$),<br>4.72 (s, 2H, $SCH_2CO$),<br>4.79 (m, 2H, $CH_2-CH=CH_2$),<br>5.35-5.45 (m, 2H, $CH=CH_2$),<br>5.9-6.1 (m, 1H, $CH_2-CH=CH_2$). | $C_9H_{10}N_6O_3S$ (282.3)<br>Ber.: C 38.30 H 3.57 N 29.77 S 11.36<br>Gef.: C 38.6 H 3.7 N 29.5 S 11.2 |
| 27 | $N_3$ | $-CH_2-CH=CH_2$ | 34<br>68.4[a)] | 0.39 (95:5) | 2135 ($N_2$)<br>2110 ($N_3$)<br>1710<br>1660 | 4.40 (s, 2H, $N_3CH_2CO$),<br>4.74 (m, 2H, $CH_2-CH=CH_2$),<br>5.3-5.4 (m, 2H, $CH=CH_2$),<br>5.85-6.0 (m, 1H, $CH_2-CH=CH_2$). | |

Fortsetzung Tabelle 2:

| Herst. beisp. No | $R^3$ | $R^4$ | Ausb. % | Rf (Tol.:Ethylac.) | IR ($cm^{-1}$) | $^1$H-NMR- (200 MHz, $CDCl_3$) $\delta$ ppm | Analyse |
|---|---|---|---|---|---|---|---|
| 28 | NCS- | $-CH_2-CH=CH_2$ | 76[b)] | 0.29 (95:5) | 2146 ($N_2$, SCN)<br>1713<br>1655 | 4.33 (s, 2H, $NCSCH_2CO$),<br>4.77 (m, 2H, $\underline{CH_2}-CH=CH_2$),<br>5.3-5.4 (m, 2H, $CH=\underline{CH_2}$),<br>5.9-6.05 (m, 1H, $CH_2-\underline{CH}=CH_2$). | $C_8H_7N_3O_3S$ (225.2)<br>Ber.: C 42.66 H 3.13 N 18.66 S 14.23<br>Gef.: C 42.9 H 3.1 N 18.7 S 14.1 |
| 29 | $CH_2=CH-CH_2-OOC-$ | $-CH_2-CH=CH_2$ | 30 | 0.31 (95:5) | 2147 ($N_2$)<br>1718<br>1655 | 3.90 (s, 2H, $COCH_2CO$),<br>4.64 (m, 2H, $\underline{CH_2}-CH=CH_2$),<br>4.72 (m, 2H, $\underline{CH_2}-CH=CH_2$),<br>5.2-5.4 (m, 4H, $CH=\underline{CH_2}$),<br>5.85-6.0 (m, 2H, $\underline{CH}=CH_2$). | $C_{11}H_{12}N_2O_5$ (252.2)<br>Ber.: C 52.38 H 4.80 N 11.11<br>Gef.: C 52.4 H 4.9 N 11.0 |
| 30 | $(C_2H_5O)_2P(=O)-$ | $-CH_2-CH=CH_2$ | 67 | 0.35 (0:1) | 2150 ($N_2$)<br>1715<br>1645<br>1255 (P=O) | 1.34 (t, J=6 Hz, 6H, $\underline{CH_3}CH_2$),<br>3.70 (d, J=23 Hz, 2H, $\underline{PCH_2}CO$),<br>4.16 (q, J=6 Hz, 4H, $CH_3\underline{CH_2}$),<br>4.75 (m, 2H, $-\underline{CH_2}-CH=CH_2$),<br>5.3-5.45 (m, 2H, $-CH=\underline{CH_2}$),<br>5.9-6.05 (m, 1H, $-CH_2-\underline{CH}=CH_2$). | $C_{11}H_{17}N_2O_6P$<br>Ber.: C 43.43 H 5.63 N 9.21 P 10.18<br>Gef.: C 43.6 H 5.6 N 9.4 P 9.7 |
| 31 | PhS | $-CH_2-CH=CH_2$ | 90[c)] | 0.55 (95:5) | 2150 ($N_2$)<br>1715<br>1650 | 4.18 (s, 2H, $SCH_2CO$),<br>4.78 (m, 2H, $\underline{CH_2}-CH=CH_2$),<br>5.3-5.5 (m, 2H, $CH=\underline{CH_2}$),<br>5.9-6.1 (m, 1H, $CH_2-\underline{CH}=CH_2$),<br>7.3-7.6 (m, 5H, Ph). | |

a) Herst.beispiel 18 + $NaN_3$ (1.3 equiv.) in DMF 1 h 0°C
b) Herst.beispiel 18 + KSCN (1.2 equiv.) 40 h Raumtemp.
c) Herst.beispiel 18 + PhSNa (1.2 equiv.) in THF 3 h 0°C

Fortsetzung Tabelle 2

| Herst. beisp. No. | R³ | R⁴ | Ausb. % | (Tol.:Ethylac.) | IR ($cm^{-1}$) | ¹H-NMR (300 MHz, $CDCl_3$) ppm | Analyse |
|---|---|---|---|---|---|---|---|
| 32 | $CH_3$- | 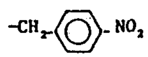 | 71 | 0.56 (4:1) | 2135 ($N_2$)<br>1713<br>1659<br>1519 ($NO_2$ as.)<br>1352 ($NO_2$ s.) | 1.14(t, J=7.5 Hz, 3H, $CH_3CH_2$), 2.86 (q, J=7.5 Hz, 2H, $CH_3CH_2$), 5.34 (s, 2H, $CO_2CH_2$), 7.55 (d, J=9 Hz, 2H, Harom.), 8.25 (d, J=9 Hz, 2H, Harom.). | $C_{12}H_{11}N_3O_5$ (277.2)<br>Ber.: C 51.99<br>H 4.00<br>N 15.16<br>Gef.: C 52.1<br>H 4.0<br>N 15.2 |
| 33 | 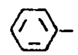 | 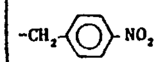 | 61 | 0.40 (95:5) | 2133 ($N_2$)<br>1720<br>1655<br>1522 ($NO_2$ as.)<br>1350 ($NO_2$ s.) | 4.19 (s, 2H, $PhCH_2$), 5.38 (s, 2H, $COOCH_2$), 7.2-7.5 (m, 5H, Ph), 7.52 (d, J=9 Hz, 2H, Harom.), 8.25 (d, J=9 Hz, 2H, Harom.), | (Schmp.: 74-76°C) |
| 34 | 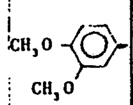 | 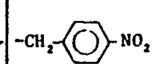 | 84 | 0.30 (4:1) | 2155 ($N_2$)<br>1715<br>1658<br>1516 ($NO_2$ as)<br>1349 ($NO_2$ s.) | 3.81,3.82 (s, 6H, $OCH_3$), 4.03 (s, 2H, $ArCH_2CO$), 5.33 (s, 2H, $COOCH_2$), 6.76 (m, 3H, Harom.), 7.48 (d, J=9 Hz, 2H, Harom.), 8.18 (d, J=9 Hz, 2H, Harom.). | $C_{19}H_{17}N_3O_7$ (399.4)<br>Ber.: C 57.14<br>H 4.29<br>N 10.52<br>Geb.: C 56.7<br>H 4.4<br>N 10.1 |
| 34 (a) | $CF_3$ | $-CH_2-CH=CH_2$ | 77 | 0.57 (9:1) | 2135 ($N_2$)<br>1710<br>1655 | 3.79 (q, J=10 Hz, 2H, $CF_3CH_2$), 4.76 (d, J=6 Hz, 2H, $-CH_2-CH=CH_2$) 5.3-5.45 (m, 2H, $-CH=CH_2$), 5.85-6.05 (m, 1H, $-CH_2-CH=CH_2$). | |

B) Beispiele zur Herstellung von Verbindungen der Formel I nach dem erfindungsgemäßen Verfahren

Beispiel 1

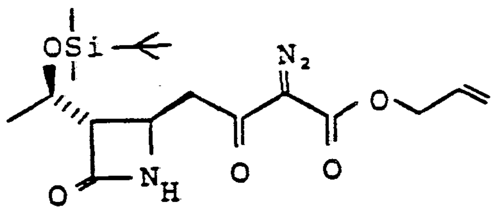

(3S,4R)-3-[(1R)-1-tert.Butyldimethylsilyloxyethyl]-4-[3-allyloxcarbonyl-3-diazo-2-oxo-propyl]-azetidin-2-on

Eine auf 0°C gekühlte Lösung von 5,75 g (20 mmol) -(3R, 4R)-4-Acetoxy-3-[(1R)-1-tert.butyldimethylsilyloxyethyl]-azetidin-2-on [S. Oida et. al. Chem. Pharm. Bull 29 2899 (1981); M. Shiozaki et al. Tetrahedron 39 2399 (1983)] und 4,37 g(26 mmol -1,3 equiv.) Allyl-2-diazo-3-oxo-butanoat in 190 ml wasserfreiem Dichlormethan werden mit 7.2 ml (52 mmol -2,6 equiv.) Triethylamin und dann tropfenweise mit 9,3 ml (48 mmol -2,4 equiv.) Trimethylsilyltrifluormethansulfonat versetzt. Das Kühlbad wurde entfernt, man rührte 1 h bei Raumtemperatur, gab 1,55 ml (8 mmol -0,4 equiv.) Trimethylsilyltrifluormethansulfonat zu und rührte weitere 0,5 h bei Raumtemperatur. Danach goß man die Reaktionslösung in ein Gemisch aus kalter gesättigter $NaHCO_3$-Lösung und Ethylacetat, extrahierte mit Ethylacetat, wusch die vereinigten organischen Extrakte mit gesättigter NaCl-Lösung und trocknete über $MgSO_4$. Das Lösungsmittel wurde im Vakuum abgedampft und der Rückstand an 150 g Kieselgel -(Toluol:Ethylacetat 4:1) filtriert. Man erhielt 6,4 g (81 %) der Titelverbindung als helle Kristalle, Schmelzpunkt: 66°C, Rf: 0,33 (Toluol:Ethylacetat 7:3) $[\alpha]_D^{20}$ = 59,2° (c 0,969, $CHCl_3$).

IR ($CHCl_3$) 2144 ($N_2$), 1761 (C=O), β-Lactam), 1714, 1649 $cm^{-1}$.

$^1$H-NMR (250 MHz, $CDCl_3$, δ 0,06 (s, 6H, $CH_3Si$), 0,88 (s, 9H, $CH_3C$-Si), 1,23 (d, J=6 Hz, 3H, $\underline{CH_3}CH$), 2,90 (dd, J=2 Hz, 5 Hz, 1H, H-3), 3,03 (dd, J=10 Hz, 17,5 Hz, 1H, $CH_2CO$), 4,06 (ddd, J=3 Hz, 5 Hz, 10 Hz, 1H, H-4), 4,24 (dq, J=5 Hz, 6 Hz, 1H, $CH_3\underline{CH}$), 4,78 (m, 2H, $\underline{CH_2}$-CH=CH), 5,3-5,5 (m, 2H, CH=$\underline{CH_2}$), 5,9-6,1 (m, $CH_2$-$\underline{CH}$=$CH_2$), 6,07 (bs, NH) zusammen 2H.

| | | | | |
|---|---|---|---|---|
| $C_{18}H_{29}N_3O_5Si$ (395,5) Ber.: | C 54,66 | H 7,39 | N 10,62 |
| Gef.: | C 54,3 | H 7,4 | N 10,4 |

Beispiel 2

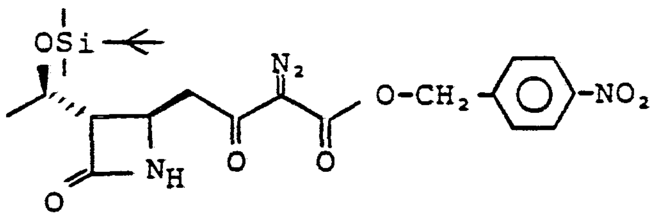

(3S,4R)-3-[(1R)-1-tert.Butyldimethylsilyloxyethyl]-4-[3-diazo-3-p-nitrobenzyloxycarbonyl-2-oxo-propyl]-azetidin-2-on

Wie für Beispiel 1 beschrieben erhielt man aus 1,44 g (5,0 mmol) (3R,4R)-4-Acetoxy-3-[(1R)-tert.butyldimethylsilyloxyethyl]-azetidin-2-on und 1,71 g (6,5 mmol) 4-Nitrobenzyl-2-diazo-3-oxo-butanoat nach Chromatographie des Rohprodukts an 35 g Kieselgel -(Toluol:Ethylacetat 7:3) 2.02 g (82%) der Titelverbindung als viskoses Öl. Rf: 0,35 (Toluol:Ethylacetat 3:2), $[\alpha]_D^{20}$ = 44.75°(c 0.373. $CHCl_3$).

IR ($CHCl_3$) 2160 ($N_2$). 1759 (C=O, β-Lactum). 1722. 1655. 1520 ($NO_2$ as.). 1355 $cm^{-1}$ ($NO_2$ s.)

$^1$H-NMR (250 MHz. $CDCl_3$) δ 0,006, 0,007 (s. 6H, $CH_3Si$), 0,86 (s. 9H, $CH_3C$-Si), 1,22 (d, J=6 Hz, 3H, $CH\underline{CH_3}$). 2,97 (dd, J=2 Hz, 5 Hz, 1H, H-3), 3,01 (dd, J=10 Hz, 17 Hz, 1H, $CH_2CO$), 3,41 (dd, J=3 Hz. 17 Hz. 1H, $CH_2CO$). 4.04 (m. 1H. H-4). 4.20 (dq, J=5 Hz. 6 Hz. 1H, $CH_3\underline{CH}$). 5,38 (s. 2H. $COOCH_2$) 7.57 und 8.28 (d, J=9 Hz. je 2H, p-$NO_2$-$C_6H_4$).

Beispiel 3

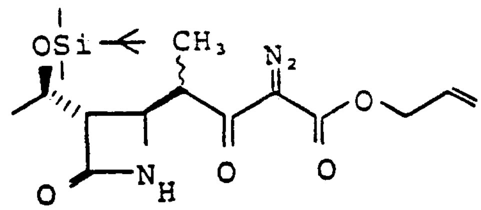

(3S.4R)-3-[(1R)-1-tert. Butyldimethylsilyloxyethyl]-4-[(1R.S)-3-allyloxycarbonyl-3-diazo-1-methyl-2-oxo-propyl]-azetidin-2-on

Eine auf 0°C gekühlte Lösung von 1.23 g (4.28 mmol) (3R,4R)-4-Acetoxy-3-[(1R)-1-tert.butyldimethylsilyloxyethyl]-azetidin-2-on und 1,18 g (6,5 mmol -1,5 equiv.) Allyl-2-diazo-3-oxo-pentanoat in 48 ml wasserfreiem Dichlormethan wurde mit 1,8 ml (13,0 mmol - 3 equiv.) Triethylamin und dann tropfenweise mit 2.3 ml (12 mmol -2.8 equiv.) Trimethylsilyltrifluormethansulfonat versetzt. Das Kühlbad wurde entfernt, man rührte 4 h bei Raumtemperatur, gab 0,4 ml (2,0 mmol -0,5 equiv.) Trimethylsilyltrifluormethansulfonat zu und rührte nochmals 1 h bei Raumtemperatur. Danach goß man die Reaktionslösung in ein Gemisch aus kalter gesättigter $NaHCO_3$-Lösung und Ethylacetrat, extrahierte mit Ethylacetat, wusch mit gesättigter NaCl-Lösung und trocknete über $MgSO_4$. Das Lösungsmittel wurde im Vakuum abgedampft und der Rückstand an 60 g Kieselgel (Toluol:Ethylacetat 4:1) chromatographiert. Man erhielt 870 mg (42 %) der Titelverbindung als 6:4 Gemisch der (1R,S)-1-Methyl Diastereomere als helle Kristalle, Schmelzpunkt: 167°C, Rf: 0,21 - (Toluol:Ethylacetat 4:1).

IR (KBr) 2135 ($N_2$), 1761 (C=O, β-Lactam), 1720 - (C=O), 1657 $cm^{-1}$.

$^1$H-NMR (250 MHz. $CDCl_3$) δ 0,07, 0,08 (s. 6H, $CH_3Si$), 0,86, 0,87 (s. 9H, $CH_3C$-Si), 1,18, 1,20 (d, J=7 Hz-3H, $\underline{CH_3}$CH), 1,22, 1,26 (d, J=7, 5 Hz, 3H, $\underline{CH_3}$CHOSi), 2,82, 2,98 (dd, J=2 Hz, 5Hz, 1H, H-3), 3,58 3,90 (m, 1H, $CH_3CH$), 3,92 (m, 1H, H-4), 4,20 (m, 1H, $CH_3\underline{CH}$OSi), 4,75 (m, 2H, $\underline{CH_2}$-CH=$CH_2$), 5,3-5,4 (m, 2H, CH=$\underline{CH_2}$), 5,81, 5,91 (bs, 1H, NH), 5,9-6,1 (m, 1H, -$CH_2$-$\underline{CH}$=$CH_2$).

Beispiel 4

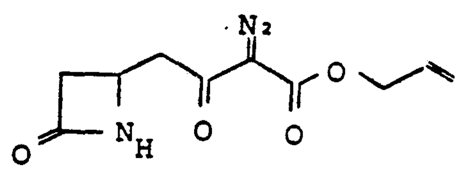

4-(3-Allyloxycarbonyl-3-diazo-2-oxopropyl)-azetidin-2-on

Wie für Beispiel 1 beschrieben erhielt man aus 2.58 g (20.0 mmol) 4-Acetoxy-2-azetidin (Deutsche Patentschrift 19 06 401 -08.02.1969] und Chromatographie des Rohprōduktes an 200 g Kieselgel (Toluol:Ethylacetat 1:3) 3,60 g - (76 %) der Titelverbindung als helle Kristalle. Schmelzpunkt : 83°C, Rf: 0.26 (Toluol:Ethylacetat 1:4).

IR (KBr) (2150 ($N_2$). 1771 (C=O. β-Lactam). 1701. 1634 $cm^{-1}$.

$^1$H-NMR (250 MHz, $CDCl_3$) δ 2.69 (dd. J=1 Hz. 14,5 Hz. 1H. H-3β). 3,04 (dd. J=10 Hz, 18 Hz, 1H, $CH_2CO$). 3.19 (ddd. J=1 Hz. 5 Hz 14.5 Hz, 1H, H-3 α). 3,40 (dd. J=5 Hz. 18 Hz, 1H. $CH_2CO$), 4,01 (m. 1H, H-4). 4,76 (m. 2H, $\underline{CH_2}$-CH=$CH_2$). 5,3-5.4 (m. 2H, -CH= $\underline{CH_2}$). 5.9-6.1 - (m. 1H. $CH_2$-$\underline{CH}$=$CH_2$). 6,10 (bs. 1H, NH).

| | | | | | |
|---|---|---|---|---|---|
| $C_{10}H_{11}N_3O_4$ | (237.2) | Ber.: | C 50.63 | H 4.67 | N 17.71 |
| | | Gef.: | C 50,2 | H 4.7 | N 16.8 |

Beispiel 5

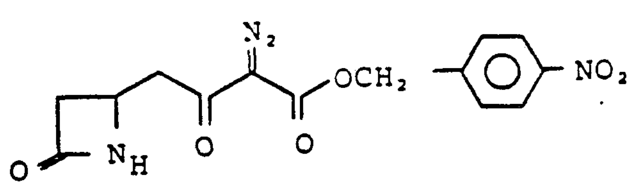

4-(3-Diazo-3-p-nitrobenzyloxycarbonyl-2-oxo-propyl)-azetidin-2-on

a) Wie für Beispiel 1 beschrieben erhielt man aus 646 mg (5,0mmol) 4-Acetoxy-2-azetidinon und 1,71 g (6,5 mmol) 4-Nitrobenzyl-2-diazo-3-oxo-butanoat und Chromatographie des Rohprodukts an 27 g Kieselgel (Toluol:Ethylacetat 3:2), 1,12 g (67 %) der Titelverbindung als farblose Kristalle, Schmelzpunkt: 104°C, Rf: 0,29 (Ethylacetat).

IR (KBr) 2145 ($N_2$), 1767 (C=O, β-Lactam, 1969, 1653, 1528 ($NO_2$ as.), 1349 $cm^{-1}$ ($NO_2$ s.).

$^1$H-NMR (250 MHz, $CDCl_3$) δ 2,70 (dd, J=1 Hz, 17,5 Hz, 1H, H-3 β), 3,08 (dd, J=9,5 Hz, 18 Hz, 1H, $CH_2CO$), 3,20 (ddd, J=1,5 Hz, 5 Hz, 17,5 Hz, 1H, H-3α), 3,40 (dd, J=5 Hz, 18 Hz, 1H, $CH_2CO$), 4,04 (m, 1H, H-4), 5,42 (s, 2H, $COOCH_2$), 7,59 und 8,42 (d, J=10 Hz, je 2H, p-$NO_2$-$C_6H_4$)

$C_{14}H_{12}N_4O_6$ (332,3) Ber.: C 50,61 H 3,64 N 16,86
Gef.: C 50,6 H 3,6 N 16,7

b) Eine auf 0°C gekühlte Lösung von 646 mg (5,0 mmol) 4-Acetoxy-2-azetidinon und 1,71 g (6,5 mmol) 4-Nitrobenzyl-2-diazo-3-oxo-butanoat in 48 ml wasserfreiem THF wurde mit 2,32 ml (13,0 mmol) Ethyldiisopropylamin und dann mit 2,3 ml (12 mmol) Trimethylsilyltrifluormethansulfonat versetzt. Das Kühlbad wurde entfernt und man rührte 1 h bei Raumtemperatur, gab 0,3 ml (1,5 mmol) Trimethylsilyltrifluormethansulfonat zu, rührte 1 h bei Raumtemperatur und arbeitete wie in Beispiel 1 beschrieben auf. Nach Chromatographie des Rohprodukts an 90 g Kieselgel (Ethylacetat) erhielt man 950 mg (57 %) der Titelverbindung als farblose Kristalle, Schmelzpunkt 103-104°C. Die weiteren physikalischen Daten waren identisch mit der nach Methode a) erhaltenen Substanz.

Beispiel 6

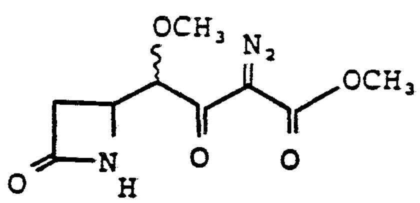

4-(3-Diazo-1-methoxy-3-methoxycarbonyl-2-oxopropyl)-azetidin-2-on

Wie für Beispiel 1 beschrieben erhielt man nach 4 h bei Raumtemperatur aus 646 mg (5,0 mmol) 4-Acetoxy-2-azetidinon und 1,12 g (6,5 mmol) Methyl-2-diazo-4-methoxy-3-oxobutanoat und Chromatographie des Rohprodukts an 60 g Kieselgel (Toluol:Ethylacetat 1:9) die Titelverbindung als nicht trennbares Diastereomerengemisch 5:1, Rf: 0,19 (Toluol:Ethylacetat 1:9).

IR ($CHCl_3$) 2150 ($N_2$), 1560 (C=O), β-Lactam 1720 1660 $CM^{-1}$.

$^1$H-NMR (250 MHz, $CDCl_3$) überwiegendes Diastereomeren δ=$CH_2$). 6,10 (bs. 2,93 (dd, J=4,5 Hz, 15 Hz, 1H, H-3), 3,07 (dd, J=1,5 Hz, 15 Hz, 1H, H-3 β), 3,46 (s, 3H, $OCH_3$), 3,90 (s, 3H, $COOCH_3$), 3,95 (m, 1H, H-4), 4,90 (d, J=4,5 Hz, 1H, CH-CH-$OCH_3$), 6,00 (bs, 1H, NH).

Beispiel 7

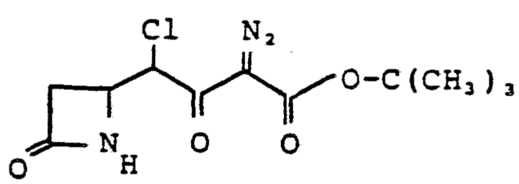

4-(3-tert.Butyloxycarbonyl-1-chlor-3-diazo-2-oxopropyl)-azetidin-2-on

Eine auf 0°C gekühlte Lösung von 646 mg (5,0 mmol) 4-Acetoxy-2-azetidinon und 1,42 g (6,5 mmol) tert. Butyl-4-chloro-2-diazo-3-oxobutanoat in 45 ml wasserfreiem Dichlormethan wurde mit 1,8 ml (13,0 mmol -2,6 equiv.) Triethylamin und dann mit 2,3 ml (12,0 mmol - 2,4 equiv.) Trimethylsilyltrifluormethansulfonat versetzt. Das Kühlbad wurde entfernt und man rührte 5 h bei Raumtemperatur, gab 0,3 ml (1,5 mmol -0,3 equiv.) Trimethylsilyltrifluormethansulfonat zu und rührte 1 h bei Raumtemperatur. Zur Aufarbeitung goß man in ein Gemisch aus kalter gesättigter $NaHCO_3$-Lösung und Ethylacetat, extrahierte mit Ethylacetat, wusch die organischen Extrakte mit gesättigter NaCl-Lösung und trocknete über $MgSO_4$. Nach Abdampfen des Lösungsmittels im Vakuum und Chromatographie an 40 g Kieselgel (Toluol:Ethylacetat 1:1) erhielt man 426 mg (30 %) der Titelverbindung als Diastereomerengemisch (10:1), Rf: 0,35 (Toluol:Ethylacetat 1:1).

IR ($CHCl_3$) 3419 (NH), 2143 ($N_2$), 1770 (C=O, $\beta$-Lactam), 1711, 1648 $cm^{-1}$.

$^1$H-NMR (250 MHz, $CDCl_3$) $\delta$ 1,56 (s, 9H, $C(CH_3)_3$), 2,95 (dd, J=1, 5Hz, 15 Hz, 1H, H-3$\beta$), 3,24 (ddd, J=2,5 Hz, 5 Hz, 15,5 Hz, 1H, H-3$\alpha$), 4,15 (m, 1H, H-4), 5,20 (d, J=9 Hz, 1H, CH-<u>CH</u>Cl), 6,05 (bs, 1H, NH).

$C_{11}H_{14}ClN_3O_4$ (287,7) Ber.: C 45,92 H 4,91

Gef.: C 46,2 H 5,1

Beispiel 8

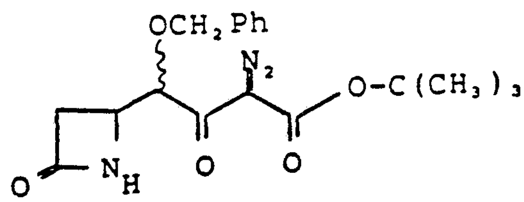

4-(1-Benzyloxy-3-tert.butoxycarbonyloxy-3-diazo-2-oxo-propyl)-azetidin-2-on

Wie für Beispiel 7 beschrieben erhielt man aus 646 mg (5,0 mmol) 4-Acetoxy-2-azetidinon und 1,89 g (6,5 mmol) tert.Butyl-4-benzyloxy-2-diazo-3-oxo-butanoat und Chromatographie des Rohprodukts an 150 g Keiselgel die Titelverbindung als Gemisch der beiden Diastereomeren im Verhältnis > 9:1, Rf: 0,56 (Toluol:Ethylacetat 1:9).

IR ($CHCl_3$) 2145 ($N_2$), 1765 (C=O, $\beta$-Lactam), 1705, 1650 $cm^{-1}$.

$^1$H-NMR (250 MHz, $CDCl_3$) $\delta$ 1,50 (s, 9H, $C(CH_3)_3$), 2,90 ddd, J=2 Hz, 5 Hz, 16 Hz, 1H, H-3$\alpha$), 3,09 (dd, J=1 Hz, 16 Hz, 1H, H-3$\beta$), 3,92 (m, 1H, H-4), 4,48, 4,72 (AB, J=11 Hz, 2H, $OCH_2Ph$), 5,08 (d, J=4,5 Hz, 1H, CH<u>CH</u>$OCH_2Ph$), 6,03 (bs, 1H, NH).

Beispiel 9

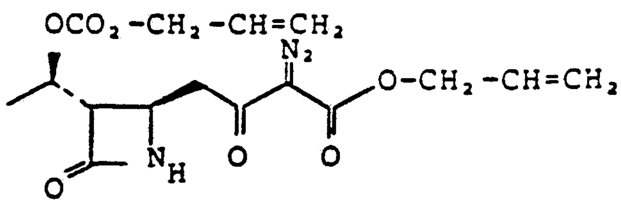

(3S.4R)-3-[(1R)-1-Allyloxycarbonyloxyethyl]-4-[3-allyloxycarbonyl-3-diazo-2-oxopropyl]-azetidin-2-on

Wie für Beispiel 1 beschrieben erhielt man aus 447 mg (1.74 mmol) (3R.4R)-4-Acetoxy-3-[(1R)-1-allyloxycarbonyloxyethyl]-azetidin-2-on und 380 mg (2,26 mmol) Allyl-2-diazo-3-oxo-butanoat nach Chromatographie des Rohprodukts an 30 g Kieselgel (Toluol:Ethylacetat 3:2) 226 mg (36 %) der Titelverbindung als Öl, Rf: 0.26 - (Toluol:Ethylacetat 3:2).

IR ($CHCl_3$) 2150 ($N_2$). 1755 (C=O, β-Lactam), 1720, 1645 $cm^{-1}$.

$^1$H-NMR (250 MHz. $CDCl_3$) δ 1,44 (d, J=6,5 Hz. 3H. $\underline{CH_3}CH$). 3,03 (dd, J=9,5 Hz, 18 Hz, $CH_2CO$), 3,08 (m. H-6) zusammen 2H, 3,44 (dd, J=4,5 Hz, 18 Hz, 1H, $CH_2CO$). 4,0 (m. 1H, H-5), 4.62 (m, 2H. -$\underline{CH_2}$-CH=$CH_2$). 4,74 (m. 2H -$\underline{CH_2}$-CH=$CH_2$). 5.11 (dq, J=6.5 Hz. 7 Hz, 1H. $CH_3C\underline{H}O$), 5,25-5,4 (m, 4H, CH=$\underline{CH_2}$), 5,9-6,0 (m, 2H, C$\underline{H}$=$CH_2$), 6,14 (bs. 1H, NH).

Beispiel 10

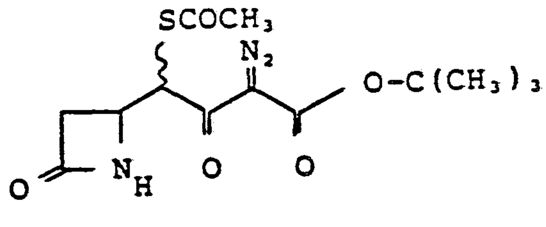

4-(1-Acetylthio-3-tert.butoxycarbonyl-3-diazo-2-oxopropyl)-azetidin-2-on

Wie für Beispiel 7 beschrieben erhielt man aus 646 mg (5,0 mmol) 4-Acetoxy-2-azetidinon und 1,67 g (6,5 mmol) tert.butyl-4-acetylthio-2-diazo-3-oxo-butanoat und Chromatographie des Rohprodukts an 50 g Kieselgel - (Toluol:Ethylacetat 3:2) 348 mg (21 %) der Titelverbindung als Gemisch der beiden Diastereomeren im Verhältnis 2:1, Rf: 0,22 (Toluol: Ethylacetat 3:2).

IR ($CHCl_3$) 2140 ($N_2$), 1765 (C=O, β-Lactam), 1713 1641 $cm^{-1}$.

$^1$H-NMR (250 MHz, $CDCl_3$) δ 1,52, 1,53 (s, 9H, C-$(CH_3)_3$), 2,38 (s, 3H, $COCH_3$), 2,7-3,1 (m, 2H, H-3, H-3'), 4,10 (m, 1H, H-4), 5,45 (d, J=6 Hz), 5,58 (d, J=5 Hz, CH-$\underline{CH}SCOCH_3$ zusammen 1 H, 5,92, 6,10 (bs, 1H, NH).

Beispiel 11

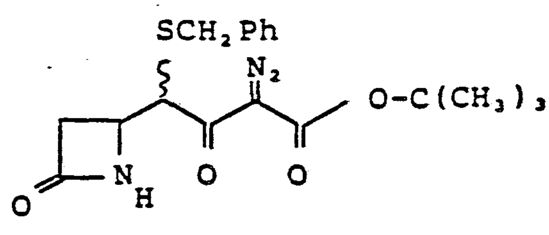

4-(1-Benzylthio-3-tert.butoxycarbonyl-3-diazo-2-oxopropyl)-azetidin-2-on

Wie für Beispiel 7 beschrieben erhielt man aus 646 mg (5,0 mmol) 4-Acetoxy-2-azetidinon und 1,99 g (6,5 mmol) tert. Butyl-4-benzylthio-2-diazo-3-oxo-butanoat nach Chromatographie des Rohprodukts an 180 g Kieselgel - (Toluol:Ethylacetat 3:2) 410 mg (22 %) der Titelverbindung als Gemisch der beiden Diastereomeren im Verhältnis 3:1, Kristalle, Schmelzpunkt: 119°C, Rf: 0,31 (Toluol: Ethylacetat 3:2).

IR($CHCl_3$) 2140 ($N_2$), 1758 (C=O, β-Lactam), 1705, 1632 $cm^{-1}$.

$^1$H-NMR (250 MHz, $CDCl_3$) δ 1,56, 1,57 (s, 9H, C-$(CH_3)_3$), 2,52 (dd, J=15 Hz, 1,5 Hz, 1H, 3-Hβ), 2,92 (ddd, J=15 Hz, 4,5 Hz, 3 Hz, 1H, 3-Hα), 3,78, 3,89 (AB, J=14,5 Hz, 2H, $SCH_2Ph$), 4,0 (m, 1H, H-4), 4,47 (d, J=10 Hz, $\underline{CH}SCH_2Ph$), 4,63 (d, J=8 Hz, $\underline{CH}SCH_2Ph$), zusammen 1H, 5,62, 5,70 (bs, 1H, NH), 7,4 (m, 5H, Ph).

| | | | | | |
|---|---|---|---|---|---|
| $C_{18}H_{21}N_3O_4S$ | Ber.: | C 57,58 | H 5,64 | N 11,19 | S 8,54 |
| | Gef.: | C 57,2 | H 5,6 | N 10,7 | S 8,7 |

Beispiel 12

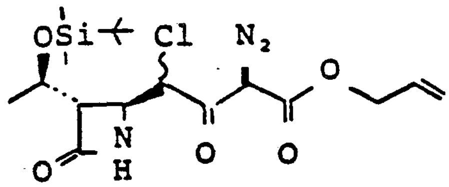

(3S.4S)-3-[(1R)-1-tert.Butyldimethylsilyloxyethyl]-4-[(1R)-3-allyloxycarbonyl-1-chlor-3-diazo-2-oxopropyl]azetidin-2-on

und

(3S.4S)-3-[(1R)-1-tert.Butyldimethylsilyloxyethyl]-4-[(1S)-3-allyloxycarbonyl-1-chlor-3-diazo-2-oxopropyl]azetidin-2-on

Wie für Beispiel 7 beschrieben erhielt man aus 1.44 g (5.0 mmol) (3R.4R)-4-Acetoxy-3-[(1R)-1-tert.butyldimethylsilyloxyethyl]-azetidin-2-on und 1.32 (6.5 mmol) Allyl-4-chlor-2-diazo-3-oxo-butanoat nach Chromatographie des Rohproduktes an 240 g Kieselgel - (Toluol:Ethylacetat 85:15) 171 mg (8 %) des unpolaren Isomere als farblose Kristalle. Schmelzpunkt: 104°C. Rf: 0.38 (Toluol:Ethylacetat 4:1).

IR (KBr) 2145 ($N_2$), 1769 (C=O, β-Lactam).1720. 1662 $cm^{-1}$.

$^1$H-NMR (300 MHz, $CDCl_3$) δ 0,08 (s, 6H, $CH_3Si$), 0,87 (s, 9H, $CH_3C$-Si), 1,20 (d, J=6,3 Hz, 3H, $\underline{CH_3}CH$), 3,07 (m, 1H, H-3), 4,08 (dd, J=2,3 Hz, 4,3 Hz, 1H, H-4), 4,17 (dq, J=3,8 Hz, 6,3 Hz, 1H, $CH_3\underline{CH}OSi$), 4,70 (d, J=5,8 Hz, 2H, $\underline{CH_2}$-CH=$CH_2$), 5,3-5,4 (m, 2H, CH=$\underline{CH_2}$), 5,50 (d, J=4,3 Hz, 1H, CHCl), 5,8-6,0 (m, 1H, -$CH_2$-$\underline{CH}$-$CH_2$), 6,0 (bs, 1H, NH).

Außerdem erhielt man 755 mg (35 %) des polaren Isomers als farblose Kristalle, Schmelzpunkt: 107°C, Rf: 0,27 (Toluol:Ethylacetat 4:1).

IR (KBr) 2140 ($N_2$), 1768 (C=O, β-Lactam), 1720, 1671 $cm^{-1}$.

$^1$H-NMR (300 MHz, $CDCl_3$) δ 0,08, 0,09 (s, 6H, $CH_3Si$), 0,88 (s, 9H, $CH_3C$-Si), 1,24 (d, J=6,3 Hz, 3H, $\underline{CH_3}CH$), 3,08 (dd, J=2,3 Hz, 2,4 Hz, 1H, H-3), 4,26 (dd, J=2 Hz, 8,9 Hz, H-4), 4,3 (m, $CH_3\underline{CH}$ OSi) zusammen 2H, 4,79 (m, 2H, $\underline{CH_2}$-CH=$CH_2$), 5,20 (d, J=8,9 Hz, 1H, CHCl), 5,3-5,4 (m, 2H, CH=$\underline{CH_2}$), 5,86 (bs, NH), 5,8-6,0 - (m, -$CH_2$-$\underline{CH}$=$CH_2$), zusammen 2H.

Beispiel 13

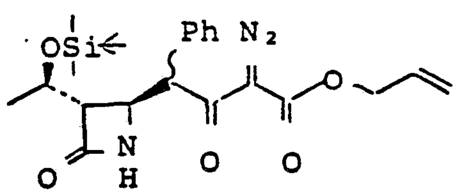

(3S,4R)-3-[(1R)-1-tert.Butyldimethylsilyloxyethyl]-4-[(1R)-3-allyloxycarbonyl-3-diazo-2-oxo-1-phenyl]propyl]azetidin-2-on

und

(3S,4R)-3-[(1R)-1-tert.Butyldimethylsilyloxyethyl]-4-[(1S)-3-allyloxycarbonyl-3-diazo-2-oxo-1-phenyl-propyl]azetidin-2-on

Wie für Beispiel 7 beschrieben erhielt man aus 1,44 g (5,0 mmol) (3R,4R)-4-Acetoxy-3-[(1R)-1-tert.butyldimethylsilyloxyethyl]azetidin-2-on und 1,59 (6,5 mmol) Allyl-2-diazo-3-oxo-4-phenylbutanoat und Chromatographie des Rohproduktes an 230 g Kieselgel - (Toluol:Ethylacetat 4:1) 200 mg (9 %) des unpolaren Titelverbindung als Öl, Rf: 0.32 (Toluol:Ethylacetat 4:1).

IR ($CHCl_3$) 2140 ($N_2$), 1760 (C=O, β-Lactam), 1720, 1650 $cm^{-}$.

1H-NMR (300 MHz, $CDCl_3$) δ 0,07, 0,08 (s, 6H, $CH_3Si$), 0,88 (s, 9H, $CH_3C$-Si), 1,19 (d, J=6,3 Hz, 3H, $\underline{CH_3}CH$), 2,68 (m, 1H, H-3), 4,18 (m, $CH_3\underline{CH}OSi$), 4,24 - (dd, J=2,5 Hz, 6,5 Hz, H-4) zusammen 2H, 4,6-4,8 (m, 2H, $\underline{CH_2}$-CH=$CH_2$), 4,97 (d, J=6,5 Hz, 1H, CHPh), 5,2-5,4 (m, 2H, CH= $\underline{CH_2}$), 5,83 (bs, NH), 5,8-6,0 (m, $CH_2$-$\underline{CH}$=$CH_2$) zusammen 2H, 7,35 (m, 5H, Ph).

Außerdem erhielt man 530 mg (22 %) des polaren Titelverbindung als farblose Kristalle, Schmelzpunkt: 139°C, Rf: 0,21 (Toluol:Ethylacetat 4:1).

IR (KBr) 2138 ($N_2$), 1764 (C=O, β-Lactam), 1725, 1658 $cm^{-1}$.

$^1$H-NMR (300 MHz, $CDCl_3$) δ 0,01 (s, 6H, $CH_3Si$), 0,25 (d, J=6 Hz, 3H, $\underline{CH_3}CH$), 0,88 (s, 9H, $CH_3C$-Si), 2,84 (m, 1H, H-3), 4,09 (m, 1H, $CH_3\underline{CH}OSi$), 4,36 (dd, J=2,3 Hz, 10,5 Hz, H-4), 4,66 (m, -$\underline{CH_2}$-CH=$CH_2$), 4,73 (d, J=10,5 Hz, CHPh) zusammen 2H, 5,2-5,4 (m, 2H, CH=$\underline{CH_2}$), 5,9-6,0 (m,$CH_2$-$\underline{CH}$=$CH_2$), 5,94 (bs, NH) zusammen 2H, 7,34 (m, 5H, Ph).

Beispiel 14

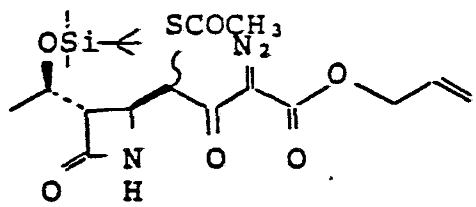

(3S,4S)-3-[(1R)-1-tert.Butyldimethylsilyloxyethyl]-4-[(1R)-1-acetylthio-3-allyloxycarbonyl-3-diazo-2-oxo-propyl]azetidin-2-on

und

(3S,4S)-3-[(1R)-1-tert.Butyldimethylsilyloxyethyl]-4-[(1S)-1-acetylthio-3-allyloxycarbonyl-3-diazo-2-oxo-propyl]azetidin-2-on

Wie für Beispiel 7 beschrieben erhielt man aus 2,70 g (9,4 mmol) (3R,4R)-4-Acetoxy-3-[(1R)-1-tert.butyldimethylsilyloxyethyl]-azetidin-2-on und 2,96 g - (12,2 mmol) Allyl-4-acetylthio-2-diazo-3-oxo-butanoat und Chromatographie des Rohproduktes an 650 g Kieselgel - (Toluol:Ethylacetat 4:1) 457 mg (15 %) des unpolaren Titelverbindung als Öl, Rf: 0,40 (Toluol: Ethylacetat 7:3).

IR ($CHCl_3$) 2135 ($N_2$), 1765 (C=O, β-Lactam), 1715, 1638 $cm^{-1}$.

$^1$H-NMR (250 MHz, $CDCl_3$) δ 0,07, 0,08 (s, 6H, $CH_3Si$), 0,87 (s, 9H, $CH_3C$-Si), 1,22 (d, J=6,5 Hz, 3H, $\underline{CH_3}CH$), 2;39 (s, 3H, $SCOCH_3$), 2,92 (m, 1H, H-3), 4,18 - (m, H-4), 4,23 (m, $CH_3\underline{CH}OSi$) zusammen 2H, 4,75 (m, 2H, -$\underline{CH_2}$-CH=$CH_2$), 5,3-5,4 (m, CH= $\underline{CH_2}$), 5,61 (d, J=4 Hz, 1H, $\underline{CH}SCOCH_3$), 5,8-6,0 (m, -$CH_2\underline{CH}$=$CH_2$), 5,98 - (bs, NH), zusammen 2H.

Außerdem erhielt man 1,5g (48 %) des polaren Titelverbindung als Öl, Rf: 0,36 (Toluol:Ethylacetat 7:3).

IR ($CHCl_3$) 3350 (NH), 2140 ($N_2$), 1758 (C=O, β-Lactam), 1715, 1640 $cm^{-1}$.

$^1$H-NMR (250 MHz, $CDCl_3$) δ 0,07 (s, 6H, $CH_3Si$), 0,88 (s, 9H, $CH_3C$-Si), 1,19 (d, J=6,5 Hz, 3H, $\underline{CH_3}CH$), 2,37 (s, 3H, $SCOCH_3$), 3,12 (m, 1H, H-3), 4,20 (dd, J=2 Hz, 7,5 Hz, H-4), 4,20 (m, $CH_3\underline{CH}OSi$) zusammen 2H, 4,75 (m, 2H, -$\underline{CH_2}$-CH=$CH_2$), 5,3-5,4 (m, 2H, -CH=$\underline{CH_2}$), 5,42 (d, J=7,5 Hz, 1H, $\underline{CH}SCOCH_3$), 5,80 (bs, 1H, NH), 5,85-6,0 (m, 1H, $CH_2$-$\underline{CH}$=$CH_2$).

Beispiel 15

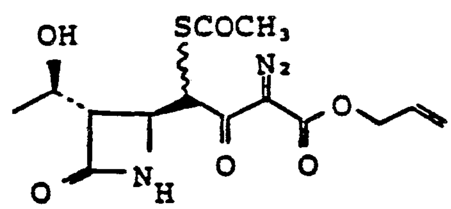

(3S,4S)-3-[(1R)-1-Hydroxyethyl]-4-[(1R,S)-1-acetylthio-3-allyloxycarbonyl-3-diazo-2-oxo-propyl]azetidin-2-on

Eine auf 0°C gekühlte Lösung von 400 mg (2,3 mmol) (3R,4R)-4-Acetoxy-3-[(1R)-1-hydroxyethyl]azetidin-2-on und 720 mg (3,0 mmol) Allyl-4-acetylthio-2-diazo-3-oxo-butanoat in 20 ml wasserfreiem Dichlormethan wurde mit 1,15 ml (8,3 mmol -3,6 equiv.) Triethylamin und dann mit 1,51 ml (7,8 mmol -3,4 equiv.) Trimethylsilyltrifluormethansulfonat versetzt. Das Kühlbad wurde entfernt, man rührte 4 h bei Raumtemperatur, gab 133 µl (0,7 mmol - 0,3 equiv.) Trimethylsilyltrifluormethansulfonat zu, rührte 3 h bei Raumtemperatur und arbeitete wie in Beispiel 1 beschrieben auf. Nach Chromatographie des Rohprodukts an 40 g Kieselgel (Ethylacetat) erhielt man die Titelverbindung als Gemisch der beiden nicht trennbaren Diasteromeren, Rf: 0.31 (Ethylacetat).

IR ($CHCl_3$) 3380 (NH. 2150 ($N_2$). 1758 (C=O, β-Lactam). 1720, 1642 $cm^{-1}$.

$^1$H-NMR (250 MHz, $CDCl_3$) δ 1,30 (d, J=6,3 Hz, 3H, $\underline{CH_3}CH$), 2,25, 2,75 (bs, 1H, OH), 2,40 (s, 3H, $SCOCH_3$), 2,96, 3,14 (m, 1H, H-3), 4,14 (m, 2H, H-4, $CH_3\underline{CH}OH$), 5,25 (m, 2H,$\underline{CH_2}$-CH=$CH_2$), 5,3-5,4 (m, 2H, CH=$\underline{CH_2}$), 5,50 (d, J=6 Hz, $\underline{CH}SCOCH_3$), 5,45 (d, J=4,8 Hz, $\underline{CH}SCOCH_3$) zusammen 1H, 5,85-6,05 (m, 1H, $CH_2$-$\underline{CH}$=$CH_2$), 6,15 (bs, 1H, NH).

Beispiel 16

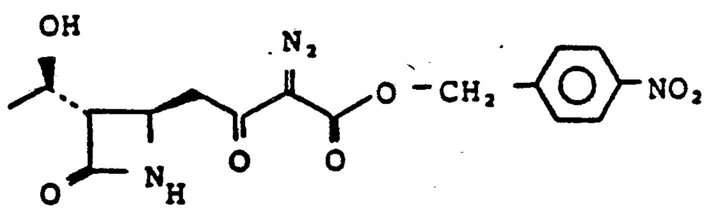

(3S.4S)-3-[(1R)-1-Hydroxyethyl]-4-[-3-diazo-3-p-nitrobenzyloxycarbonyl-2-oxopropyl]azetidin-2-on

10 mg (0.02 mmol) (3S.4R)-3-[(1R)-1-tert.Butyldimethylsilyloxyethyl]-4-[3-diazo-3-p-nitrobenzyloxycarbonyl-2-oxopropyl]azetidin-2-on wurden bei 0°C in 1 ml einer Maßlösung von 3,76µl (0.03 mmol -1,5 equiv.) Bortrifluoridetherat in Acetonitril gelöst und 2 h bei dieser Temperatur gerührt. Danach wurde die Reaktionslösung an 0.5 g Kieselgel/$NaHCO_3$ filtriert und im Vakuum eingedampft, man erhielt die Titelverbindung. Rf: 0.24 (Toluol:Ethylacetat 1:9).

IR ($CHCl_3$) 3419 (OH). 2142 ($N_2$). 1768 $cm^{-1}$ (C=O. β-Lactam).

$^1$H-NMR (250 MHz. $CDCl_3$) δ 1.35 (d. J=6.5 Hz. 3H. $\underline{CH_3}$CH). 1.62 (bs. 1H. OH). 2.91 (dd. J=1,5 Hz. 7.5 Hz. 1H. H-6). 3.22 (dd. J=7.5 Hz. 19 Hz. 1H. $CH_2$CO). 3.35 - (dd. J=7.5 Hz. 19 Hz. 1H. $CH_2$CO). 4.00 (m. 1H. H-5). 4.20 (dq. J=6,5 Hz. 7.5 Hz. 1H. $CH_3\underline{CH}$OH). 5.40 (s. 2H. $COOCH_2$). 6.00 (bs. 1H. NH). 7.58 und 8.30 (d. J=9 Hz. je 2H. p-$NO_2$-$C_6H_4$).

Die Verbindung dieses Beispiels ist als Zwischenstufe zur Synthese von Thienamycin verwendet worden (U.S. Patent 4 290 947: Th. N. Salzmann et al. J.Am.Chem.Soc. 102 6163 (1980)].

Herstellungsbeispiel 17

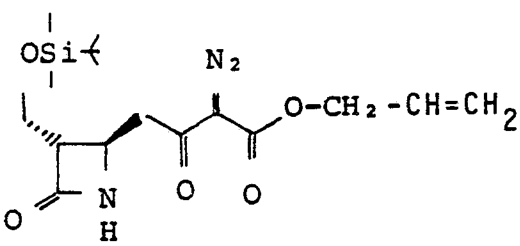

(3S, 4R)-3-[tert.Butyldimethylsilyloxymethyl]-4-[3-allyloxycarbonyl-3-diazo-2-oxo-propyl]azetidin-2-on

Eine auf 0°C gekühlte Lösung von 547 mg (2.0 mmol) (3S. 4R)-4-Acetoxy-3-[tert.butyldimethylsilyloxymethyl]azetidin-2-on und 437 mg - (2.6 mmol)Allyl-2-diazo-3-oxo-butanoat in 18 ml wasserfreiem Dichlormethan wurde mit 0.72 ml (5.2 mmol ) Triethylamin und dann tropfenweise mit 0.93 ml (4.8 mmol) Trimethylsilyltrifluormethansulfonat versetzt. Das Kühlbad wurde entfernt, man rührte 15 min bei Raumtemperatur, gab 0.12 ml (0.6 mmol) Trimethylsilyltrifluormethansulfonat zu und rührte weitere 15 min bei Raumtemperatur. Danach goß man die Reaktionslösung in ein Demisch aus kalter gesättigter $NaHCO_3$-Lösung und Ethylacetat, extrahierte mit Ethylacetat. wusch die organischen Extrakte mit gesättigter NaCl-Lösung und trocknete über $MgSO_4$. Das Lösungsmittel wurde i.Vak. abgedampft und der Rückstand an 50 g Kieselgel (Toluol: Ethylacetat 4:1) chromatographiert. Man erhielt 462 mg (61%) der Titelverbindung als Öl, Rf.: 0.11 (Toluol:Ethylacetat 4:1).

IR ($CHCl_3$) 3380 (NH), 2150 ($N_2$), 1760 (C=O, β-Lactam), 1715, 1648 $cm^{-1}$.

$^1$H-NMR (250 MHz, $CDCl_3$) δ 0.07 (s, 6H, $CH_3$Si), 0.88 (s, 9H, $CH_3$C-Si), 3.03 (m, 1H, H-3), 3.11 (dd, J=10 Hz, 18Hz, 1H, $CH_2$CO), 3.40 (dd, J=5 Hz, 18 Hz, 1H, $CH_2$Co), 3.95 (m, H-4), 4.0 (m, $CH_2$OSi) Zusammen 3H, 4.76 (m, 2H, $\underline{CH_2}$-CH=$CH_2$), 5.3-5.45 (m, 2H, -CH=$\underline{CH_2}$), 5.9-6.1 (m, -$CH_2$-$\underline{CH}$=$CH_2$), 6.01 (bs, NH) Zusammen 2H.

Beispiel 18

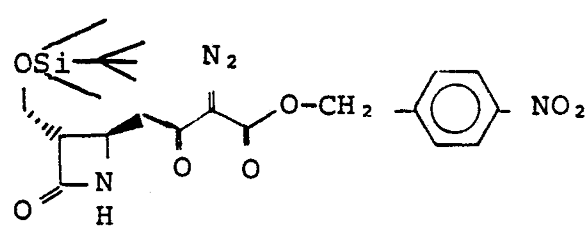

(3S, 4R)-3-[tert.Butyldimethylsilyloxymethyl]-4-[3-diazo-3-p-nitrobenzyloxycarbonyl-2-oxo-propyl]-azetidin-2-on

Wie für Beispiel 17 beschrieben erheilt man aus 930 mg (3.4 mmol) (3R, 4R)-4-Acetoxy-3-[tert.butyldimethylsilyloxymethyl]azetidin-2-on und 1.16 mg - (4.42 mmol -1.3 equiv.) 4-Nitrobenzyl-2-diazo-3-oxo-butanoat nach Chromatographie des Rohproduktes an 100 g Kieselgel (Toluol:Ethylacetat 1:1) 1.22 g (65%) der Titelverbindun als farblose Kristalle,

Schmelzpunkt: 104°C, Rf.: 0.31 (Toluol:Ethylacetat 1:1)

IR (KBr) 3352 (NH), 2139 ($N_2$), 1755 (C=O, β-Lactam), 1713, 1660, 1525 ($NO_2$ as.), 1349 $cm^{-1}$ ($NO_2$ s.).

$^1$H-NMR (300 MHz, $CDCl_3$) δ 0.06 (s, 6H, $CH_3Si$), 0.86 (s, 9H, $CH_3C$-Si), 2.98 (m, 1H, H-3), 3.05 (dd, J=9 Hz, 18 Hz, 1H, $CH_2CO$), 3.34 (dd, J=5 Hz, 18 Hz, 1H, $CH_2CO$), 3.89 (m, 1H, H-4), 3.96 (m, 2H, $CH_2OSi$), 5.31 - (s, 2H, $COOCH_2$) 5.93 (bs, 1H, NH), 7.50 (d. J=9 Hz, 2H. $H_{arom.}$), 8.23 (d, J=9 Hz, 2H, $H_{arom.}$).

Wie in Beispiel 7 beschrieben erhielt man durch Umsetzung von (3R.4R)-4-Acetoxy-3-[(1R)-1-tert.-butyldimethylsilyloxyethyl]-azetidin-2-on mit den entsprechenden Diazo-acetessigestern die folgenden Verbindungen (Beispiel 19-24, Tab. 3):

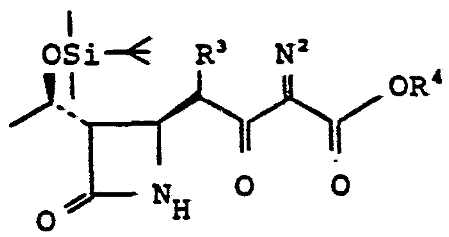

(Die Spalte Zeit steht für die Rührdauer bei Raumtemp. nach Zugabe der katalytischen Menge Trimethylsilyltrifluormethansulfonat).

Tabelle 3

| Beisp. No. | $R^3$ | $R^4$ | Zeit (h) | Rf (Tol.:Ethylac.) | IR ($cm^{-1}$) | $^1H$-NMR (250 MHz, $CDCl_3$) δ ppm | Bemerkungen |
|---|---|---|---|---|---|---|---|
| 19 | SCN | $CH_2-CH=CH_2$ | 2 | 0.14, 0.18 (4:1) | 3416 (NH)<br>2155 ($N_3$)<br>1775 (C=O)<br>1714<br>1652 | 0.08 (s, 6H, $CH_3Si$),<br>0.88 (s, 9H, $CH_3C$-Si),<br>1.23, 1.30 (d, J=6.3 Hz, 3H, $CH_3CH$),<br>3.0-3.2 (m, 1H, H-3),<br>4.25-4.45 (m, 2H, $CH_3CHO$, H-4),<br>4.68 (d, J=10 Hz, CHSCN),<br>5.19 (d, J=5.5 Hz, CHSCN), zusammen 1H,<br>4.81 (m, 2H, $CH_2-CH=CH_2$),<br>5.4-5.5 (m, 2H, $CH=CH_2$),<br>5.9-6.1 (m, 1H, $CH_2-CH=CH_2$),<br>6.18, 6.60 (bs, 1H, NH). | $C_{19}H_{28}N_4O_5SSi$ (452.61)<br>Ber.: C 50.42<br>H 6.24<br>N 12.38<br>S 7.08<br>Gef.: C 50.5<br>H 6.3<br>N 12.4<br>S 7.0<br>Diastereomere ca 3:2 |
| 20 | $N_3$ | $CH_2-CH=CH_2$ | 2 | 0.25 (4:1) | 3395 (NH)<br>2130 ($N_3$)<br>2115 ($N_3$)<br>1762 (C=O)<br>1718<br>1658 | 0.08, 0.09 (s, 6H, $CH_3Si$),<br>0.89 (s, 9H, $CH_3C$-Si),<br>1.23 (d, J=6.5 Hz, 3H, $CH_3CH$),<br>3.14 (m, 1H, H-3),<br>4.13 (dd, J=2 Hz, 9 Hz, 1H, H-4),<br>4.30 (m, 1H, $CH_3CHO$),<br>4.80 (m, 2H, $CH_2-CH=CH_2$),<br>5.03 (d, J=9 Hz, 1H, $CHN_3$),<br>5.4-5.5 (m, 2H, $CH=CH_2$),<br>5.77 (bs, 1H, NH),<br>5.9-6.1 (m, 1H, $CH_2-CH=CH_2$). | Diastereomere ca 1:1 |
| 21 | PhS | $CH_2-CH=CH_2$ | 7 | 0.23 (9:1) | 3380 (NH)<br>2140 ($N_3$)<br>1761 (C=O)<br>1645 | 0.07, 0.08 (s, 6H, $CH_3Si$),<br>0.88 (s, 9H, $CH_3C$-Si),<br>1.22, 1.26 (d, J=6.5 Hz, 3H, $CH_3CH$),<br>3.08 (m, 1H, H-3),<br>4.25 (m, 2H, H-4, $CH_3CHO$),<br>4.46 (d, J=9 Hz, CHSPh),<br>4.68 4.72 (m, 3H, CHSPh, $CH_2-CH=CH_2$) zusammen 3H,<br>5.4-5.5 (m, 2H, $CH=CH_2$),<br>5.9-6.05 (m, 1H, $CH_2-CH=CH_2$),<br>6.08, 6.15 (bs, 1H, NH). | Diastereomere |

Tabelle 3 (Fortsetzung)

| Beisp. No. | $R^1$ | $R^4$ | Zeit (h) | Rf (Tol.:Ethylac.) | IR ($cm^{-1}$) | $^1$H-NMR (250 MHz, $CDCl_3$) δ ppm | Bemerkungen |
|---|---|---|---|---|---|---|---|
| 22 | 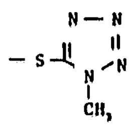 | $CH_2$-CH=$CH_2$ | 65<br>0°C | 0.29 (7:3) | 3385 (NH)<br>2145 ($N_2$)<br>1770 (C=O)<br>1718<br>1645 | 0.08, 0.09 (s, 6H, $CH_3$Si),<br>0.89 (s, 9H, $CH_3$C-Si),<br>1.29 (d, J=6.3 Hz, 3H, $CH_3$CH),<br>3.31 (m, 1H, H-3),<br>3.97 (s, 3H, $NCH_3$),<br>4.34 (m, 2H, H-4, $CH_3$CHO),<br>4.75 (m, 2H, $CH_2$-CH=$CH_2$),<br>5.3-5.5 (m, 2H, CH=$CH_2$),<br>5.9-6.1 (m, 1H, $CH_2$-CH=$CH_2$),<br>6.85 (bs, 1H, NH). | Diastereomere<br>ca. 8:1 |
| 23 | O<br>‖<br>-P(OEt)$_2$ | $CH_2$-CH=$CH_2$ | 66 | 0.06 (0:1) | 3341 (NH)<br>2140 ($N_2$)<br>1772 (C=O)<br>1715<br>1650<br>1250 | 0.07, 0.08 (s, 6H, $CH_3$Si),<br>0.88 (s, 9H, $CH_3$C-Si),<br>1.24 (d, J=6 Hz, 3H, $CH_3$CH),<br>3.1 (m, 1H, H-3),<br>4.1-4.25 (m, 2H, $CH_3$CHO, H-4),<br>4.72 (m, 2H, $CH_2$-CH=$CH_2$),<br>5.3 (dd, J=5 Hz, 22 Hz, CHP),<br>5.3-5.5 (m, CH=$CH_2$) zusammen 3H,<br>5.9-6.9 (m, 1H, $CH_2$-CH=$CH_2$),<br>6.15 (bs, 1H, NH). | Diastereomere |
| 24 | -COO-$CH_2$-CH=$CH_2$ | $CH_2$-CH=$CH_2$ | 5.5 | 0.29, 0.25<br>(4:1) | 3395 (NH)<br>2152 ($N_2$)<br>1760 (C=O)<br>1720<br>1650 | 0.05 (s, 6H, $CH_3$Si),<br>0.86, 0.87 (s, 9H, $CH_3$C-Si),<br>1.11, 1.15 (d, J=6.5 Hz, 3H, $CH_3$CH),<br>3.21, 3.24 (m, 1H, H-3),<br>4.24 (m, $CH_3$CHO),<br>4.34 (m, H-4) zusammen 2H,<br>4.66 (d, J=7 Hz, CHCOO),<br>4.74, 4.77 (m, $CH_2$-CH=$CH_2$) zus. 5H,<br>5.25-5.45 (m, 4H, CH=$CH_2$),<br>5.85-6.05 (m, $CH_2$-CH=$CH_2$),<br>5.92, 6.10 (bs, NH) zusammen 3H. | Diastereomere<br>1:1 |

| Herst. Beisp. No. | $R^3$ | $R^4$ | Zeit (h) | (Tol.:Ethylac.) | IR ($cm^{-1}$) | $^1H$-NMR (250 MHz, $CDCl_3$) ppm | Analyse |
|---|---|---|---|---|---|---|---|
| 25 | $CH_3$ | 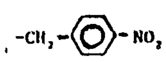 | 1,5 | 0.26 (7:3) | 3350 (NH) 2150 ($N_3$) 1775 (C=O) 1725 1525 ($NO_2$as.) 1355 ($NO_2$s.) | 0.06-0.08 (s, 6H, $CH_3Si$), 0.84, 0.86 (s, 9H, $CH_3C$-Si), 1.2 (m, $CH_3CH$), 1.22, 1.25 (d, J=7,5 Hz, $CH_3CHOSi$) zusammen 6H, 2.80, 2.96 (dd, J=15 Hz, 6 Hz, 1H, H-3), 3.55 (m, 1H, $CH_3CH$), 3.89, 3.90 (dd, J=15 Hz, 10 Hz, 1H, H-4), 4.18 (m, 1H, $CH_3CHOSi$), 5.35, 5.36 (s, 2H, $COOCH_2$), 7.54 (d, J=9 Hz, 2H, Harom.) 8.26 (d, J=9 Hz, 2H, Harom.). | Diastereomere 1'(R) : 1'(S) = 2:3 |
| 26 | 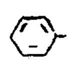 | 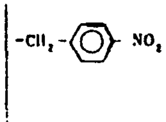 | 15 | 0.23 (7:3) | 3350 (NH) 2148 ($N_3$) 1762 (C=O) 1720 1530 ($NO_2$as.) 1555 ($NO_2$s.) | 0.01, 0.04, 0.06, 0.08 (s, 6H, $CH_3Si$), 0.25, 1.20 (d, J=6 Hz, 3H, $CH_3CHO$), 0.88 (s, 9H, $CH_3C$-Si), 2.7-3.0 (m, 1H, H-3), 3.90 (m, H-4), 4.35 (dd, J=2 Hz, 10 Hz, H-4) zusammen 1H, 4.15 (m, 1H, $CH_3CHO$), 4.68 (d, J=10 Hz, PhCH), 5.18, 5.30 (d, 2H, $COOCH_2$), 5.91 5.95 (bs, 1H, $NH_2$) 7.2 - 7.35 (m, 5H, Ph), 7.43 (d, J=9 Hz, 2H, Harom.), 8.20 (d, J=9 Hz, 2H, Harom.). | Diastereomere 45 : 55 |

C) Beispiele für die Verwendung von Verbindungen der allgemeinen Formel 1 zur Herstellung von Carbapenem-Antibiotika

Beispiel 1

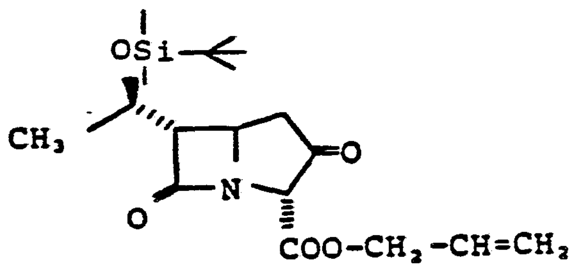

(2S,5R,6S)-2-Allyloxycarbonyl-3,7-dioxo-6-[(1R)-1-tert.-butyldimethylsilyloxyethyl]-1-azabicyclo[3,2,0]heptan

Eine Lösung von 3,96 g (10 mmol) (3S,4R)-3-[(1R)-1-tert. butyldimethylsilyloxyloxyethyl]-4-(3-allyloxycarbonyl-3-diazo-2-oxopropyl]-azetidin-2-on in 50 ml wasserfreiem Dichlormethan wurde in Gegenwart von 40 mg (1 mol %) Rhodium(II)acetat 30 Minuten im Rückfluß erhitzt. Die Reaktionslösung wurde durch wenig Kieselgur und Kieselgel filtriert und im Vakuum eingedampft. Man erhielt 3,5 g - (95 %) der Titelverbindung als farbloses Öl, Rf: 0,46 (Toluol:Ethylacetat 7:3).

IR ($CHCl_3$) 1766, 1740 $cm^{-1}$.

$^1$H-NMR (250 MHz, $CDCl_3$) δ 0,06 s, 6H, $CH_3Si$), 0,87 (s, 9H, $CH_3C$-Si), 1,26 (d, J=6,5 Hz, 3H, $\underline{CH_3}CH$), 2,44 (dd, J=8 Hz, 19 Hz, 1H, $CH_2CO$), 2,88 (dd, J=8 Hz, 19 Hz, 1H, $CH_2CO$), 3,15 (dd, J=2 Hz, 5 Hz, 1H, H-6) 4,16 (ddd, J=2 Hz, 8 Hz, 8 Hz, 1H, H-5), 4,33 (dq, J=5 Hz, 6,5 Hz, 1H, $CH_3\underline{CH}$), 4,65 (m, 2H, $\underline{CH_2}$-CH=$CH_2$), 4,71 (s, 1H, H-2), 5.25-5.4 (m, 2H, CH=$CH_2$), 5,8-6,0 (m, 1H, $CH_2$-$\underline{CH}$.$CH_2$).

Beispiel 2

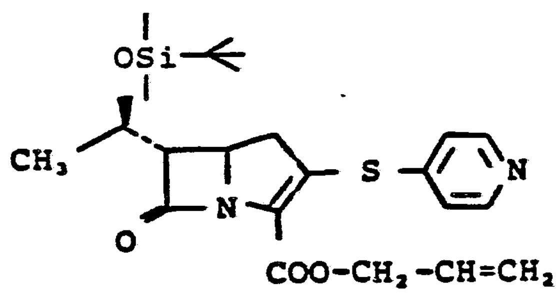

(5R,6S)-2-(4-Pyridylthio)-6-[(1R)-1-tert.butyldimethylsilyloxyethyl]-carbapen-2-em-3-carbonsäureallylester

Zu einer auf -78°C gekühlte Lösung von 3,68 g (10 mmol) (2S,5R,6S)-2-Allyloxycarbonyl-3,7-dioxo-6-[(1R)-1-tert.butyldimethylsilyloxyethyl]-1-azabicyclo[3,2,0]heptan in 100 ml wasserfreiem THF wurden 1,94 ml (13 mmol) 1,5-Diazabicyclo[5,4,0]undec-5-en (DBU) und nach 20 Minuten 2,2 ml (13 mmol) Trifluormethansulfonsäureanhydrid getropft. Man rührte 30 Minuten bei -78°C nach und konzentrierte die Lösung im Vakuum auf ca. 40 ml, ohne daß die Temperatur über -20°C anstieg. Anschließend gab man nacheinander 20 ml DMF, 2,1 ml (12 mmol) Ethyldiisopropylamin und 1,33 g (12 mmol) 4-Mercaptopyridin zu und rührte 1 h bei -20°C. Danach wurde die Mischung mit Ethylacetat verdünnt und in kalte, gesättigte $NaHCO_3$-Lösung gegossen. Man extrahierte mit Ethylacetat wusch mit gesättigter NaCl-Lösung und trocknete mit $MgSO_4$. Nach Abdampfen des Lösungsmittels im Vakuum und Chromatographie des Rückstands an 600 g Kieselgel (Toluol:Ethylacetat) erhielt man 3,1 g (66 %) der Titelverbindung als viskoses Öl, Rf: 0,30 (Toluol:Ethylacetat 7:3).

IR ($CHCl_3$) 1781 (C=O, β-Lactam), 1710 $cm^{-1}$ (C=O, Ester).

$^1$H-NMR (250 MHz, $CDCl_3$) δ 0,02, 0,04 (s, 6H, $CH_3Si$), 0,84 (s, 9H, $CH_3C$-Si), 1,10 (d, J=6,5 Hz, $C\underline{H_3}CH$), 2,86 (dd, J=11 Hz, 18 Hz, 1H, H-1), 3,04 (dd, J=9 Hz, 18 Hz, 1H, H-1'), 3,48 (m, 1H, H-6), 4,17 (m, 2H, H-5, $CH_3C\underline{H}$), 4,73 (m, 2H, $\underline{CH_2}$-CH=CH), 5,25 (dd, J=1 Hz, 10,5 Hz, 1H, CH=$\underline{CH_2}$-cis), 5,47 (dd, J = 1Hz, 17Hz, 1H, CH=$CH_2$-trans), 5,9-6,1 (m, 1H, $CH_2$-$\underline{CH}$=$CH_2$), 7,63 (d, J=5,5 Hz, 2H, 3,5-Pyridyl-H), 8,62 (d, J=5,5 Hz, 2H, 2,6-Pyridyl-H).

Beispiel 3

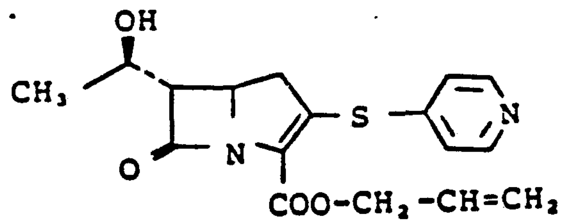

(5R,6S)-2-(4-Pyridylthio)-6-[(1R)-1-hydroxyethyl]-carbapen-2-em-3-carbonsäureallylester

Zu einer auf 0°C gekühlte Lösung von 2,97 g (6.5 mmol) (5R.6S)-2-(4-Pyridylthio)-6-[(1R)-1-tert.butyldimethylsilyloxyethyl]-carbapen-2-em-3-carbonsäureallylester in 60 ml wasserfreiem THF gab man 3.69 ml (64.5 mmol -10 equiv.) Eisessig und 19.3 ml (19.3 mmol -3 equiv.) einer 1M-Lösung von Tetrabutylammoniumfluorid in THF. Das Kühlbad wurde entfernt und die Reaktionslösung blieb 62 h bei Raumtemperatur stehen. Zur Aufarbeitung goß man in ein Gemisch aus kalter gesättigter $NaHCO_3$-Lösung und Ethylacetat. extrahierte mit Ethylacetat. wusch mit gesättigter $NaHCO_3$-und NaCl-Lösung und trocknete über $MgSO_4$. Nach Abdampfen des Lösungsmittels im Vakuum und Chromatographie des Rückstande an 360 g Kieselgel (Toluol:Ethylacetat 9:1) erhielt man 472 mg (21 %) der Titelverbindung als Öl, Rf: 0,24 (Toluol:Ethylacetat 9:1).

IR ($CHCl_3$) 3400 (OH), 1776 (C=O, $\beta$-Lactam), 1720 $cm^{-1}$ (C=O, Ester).

$^1$H-NHM (250 MHz, $CDCl_3$) $\delta$ 1,32 (d, J=6,5 Hz, 3H, $\underline{CH_3}$CH), 2,78, 2,90, (dd, J=10 Hz, 18 Hz, 2H, H-1), 3,16 (dd, J=2,5 Hz, 7 Hz, 1H, H-6), 4,2 (m, 2H, H-5), $CH_3\underline{CH}$), 4,75, 4,87 (dd, J=7,5 Hz, 14,5 Hz, 2H, $\underline{CH_2}$-CH=$CH_2$), 5,30 (d, J=9 Hz, 1H, CH=$\underline{CH_2}$-cis), 5,48 (d, J=17 Hz, 1H, CH=$\underline{CH_2}$-trans), 5,9-6,1 (m, 1H, -$CH_2$-$\underline{CH}$=$CH_2$), 7,43 (d, J=7 Hz, 2H, 3,5-Pyridyl-H), 8,64 (d, J=7 Hz, 2H, 2,6-Pyridyl-H).

Beispiel 4

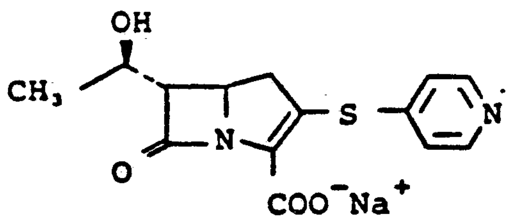

Natrium(5R,6S)-2-(4-pyridylthio)-6-[(1R)-1-hydroxyethyl]-carbapen-2-em-3-carboxylat

Eine Lösung von 462 mg (1,33 mmol) (5R,6S)-2-(4-Pyridylthio)-6-[(1R)-1-hydroxyethyl]-carbapen-2-em-3-carbonsäureallylester in 3,4 ml wasserfreiem Dichlormethan wurde nacheinander mit 2,8 ml (1,4 mmol -1,05 equiv.) 0,5 M Natrium-2-ethyl-hexanoat in Ethylacetat, 35 mg (0,13 mmol -0,1 equiv.) Triphenylphosphin und 35 mg (0,03 mmol) Tetrakis-(triphenylphosphin)-palladium (O) versetzt und 1 h bei Raumtemperatur gerührt. Anschließend wurde mit 20 ml wasserfreiem Aceton verdünnt. Der Niederschlag wurde abgesaugt, und 0,5 h mit 50 ml Ether gut durchgerührt, erneut abgesaugt und im Hochvakuum über $P_4O_{10}$ getrocknet. Man erhielt 361 mg (83 %) der Titelverbindung als farbloses hygroskopisches Pulver.

IR (KBr) 3400 (OH), 1755 (C=O, $\beta$-Lactam), 1600 - (COONa), 1576, 1392 $cm^{-1}$.

$^1$H-NMR (250 MHz, DMSO) $\delta$ 1,11 (d, J=6 Hz, 3H, $\underline{CH_3}$CH), 2,72, 2,75, (s, 2H, H-1), 3,20 (dd, J=3 Hz, 6 Hz, 1H, H-6) 3,92 (m, 1H, H-5), 4,07 (m, 1H, $CH_3\underline{CH}$), 7,33 (d, J=6 Hz, 2H, 3,5-Pyridyl-H), 8,44 (d, J=6 Hz, 2H, 2,6-Pyridyl-H).

**Ansprüche**

1. Verfahren zur Herstellung von 4-[3-Carboxy-3-diazo-2-oxopropyl]azetidin-2-onen der allgemeinen Formel I

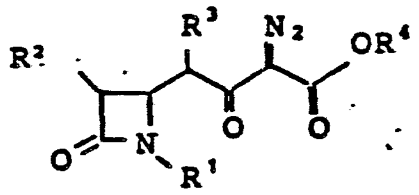

(I),

in welcher

$R^1$ -für Wasserstoff oder

-für eine Aminoschutzgruppe steht,

$R^2$ -für Wasserstoff,

-für gegebenenfalls substituiertes Alkyl,

-für gegebenenfalls substituiertes Aryl,

-für gegebenenfalls substituiertes Heterocyclyl,

-für Cyano, Azido, Halogen, oder

-für $NHR^1$ steht, wobei $R^1$ die oben angegebene Bedeutung hat,

$R^3$ -für Wasserstoff,

- für gegebenenfalls substituiertes Alkyl,

-für gegebenenfalls substituiertes Aryl.

-für gegebenenfalls substituiertes Heterocyclyl.

-für Halogen. Cyano, Cyanato, Azido. Nitro oder =N-Phenyl,

-für $-COR^5$, $-CO_2R^5$, $-CO-NR^5R^6$, $-CSNR^5R^6$, $-C(=NR^5)-NR^5R^6$,

-für $-NR^5R^6$, $-NR^5-COR^6$, $-NR^5-CO_2R^7$, $-NR^6-CR^5(=NR^6)$, $-NR^6-CO-NR^5R^6$, $-NR^5-CS-NR^5R^6$, $-NR^5-C(=NR^6)-NR^5R^6$, $-NR^5-SO_2R^7$, oder

-für $OR^5$, $-OCOR^5$, $-OCO-NR^5R^6$, $-OSO_2R^7$, $OSO_3R^5$, $-OPO(OR^5)OR^6$, oder

-für $-SR^5$, $-S(O)_nR^7$ wobei n=1 oder 2 ist, $-SCOR^5$, $-SO_2OR^5$, $-SO_2NR^5R^6$, -SCN $-SCONHR^5$ oder

-für $-PX(OR^5)OR^6$, $PX(NR^5R^6)_2$, $PX(OR^5)NR^5R^6$ wobei jeweils

$R^5$, $R^6$ -gleich oder verschieden sind und

-für gegebenenfalls substituiertes Alkyl,

-für gegebenenfalls substituiertes Aryl

-für gegebenenfalls substituiertes Heterocyclyl steht. oder

-gegebenenfalls für eine Hydroxy-, Mercapto-, Amino-oder Carboxyschutzgruppe steht.

$R^7$ -die gleiche Bedeutung hat wie $R^5$, $R^6$, jedoch nicht für Wasserstoff oder eine Schutzgruppe steht,

und

X -Sauerstoff oder Schwefel steht,

und

$R^4$ -für eine Carboxyschutzgruppe oder

-für einen in vivo spaltbaren Esterrest steht,

dadurch gekennzeichnet, daß man 4-Acetoxy-2-azetidinone der allgemeinen Formel II

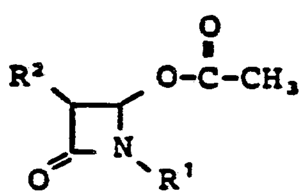

(II),

in welcher

$R^1$ und $R^2$ die oben angegebene Bedeutung haben, mit Verbindungen der allgemeinen Formel III

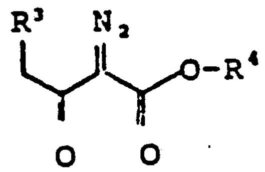

(III),

in welcher

$R^3$ und $R^4$ die oben angegebene Bedeutung haben.

in einem inerten Lösungsmittel, in Gegenwart einer Base und eines Silylierungsmittels in einem Eintopfverfahren umsetzt.

2. Verfahren gemäß Anspruch 1 zur Herstellung von Verbindungen der allgemeinen Formel I. in welcher

$R^1$ -für Wasserstoff oder

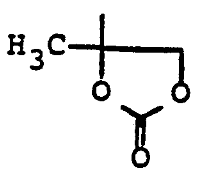

,

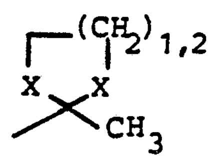

wobei X Schwefel oder Sauerstoff bedeutet oder

-für geradkettiges, verzweigtes oder cyclisches, gesättiges, oder ungesättigtes Alkyl mit bis zu 6 C-Atomen. das gegebenenfalls substituiert ist durch Fluor, Chlor, oder die Gruppe $OR^8$

wobei

$R^8$ -für Wasserstoff.

-für Trimethylsilyl, Triethylsilyl. Triisopropylsilyl, tert.-Butyldimethylsilyl. Triphenylsilyl, Trimethylsilylethoxycarbonyl.

-für Benzyl. Benzyloxycarbonyl. 2-bzw. 4-Nitrobenzyl. 2-bzw. 4-Nitrobenzyloxycarbonyl. tert.-Butyloxycarbonyl. Allyloxycarbonyl. 4-Methoxybenzyl. 4-Methoxybenzyloxycarbonyl. oder

-für Formyl. Acetyl, Trichloracetyl. Trichlorethoxycarbonyl. 2.4-Dimethoxybenzyloxycarbonyl. Methoxymethyl, Methylthiomethyl. Methoxyethoxymethyl [2-(Trimethylsilyl)ethoxy]-methyl. 2-(Methylthiomethoxy)ethoxycarbonyl, Tetrahydropyranyl, oder Benzoyl steht.

$R^3$ -für Wasserstoff,

-für gegebenenfalls durch Chlor, Nitro. Methyl. Methoxy, Methylthio. Trifluormethyl oder durch (gegebenenfalls geschütztes) Hydroxy substituiertes Phenyl,

-für Fluor. Chlor. Brom, Cyano, Cyanato oder Azido,

-für gegebenenfalls durch Methyl, Amino (gegebenenfalls geschützt), Fluor, Chlor, Trifluormethyl oder Hydroxy substituiertes Thienyl, Furyl, Imidazolyl, Thiazolyl, Thiadiazolyl, Triazolyl, Tetrazolyl, Pyridyl, Piperazinyl oder Pyrimidyl,

-für S-Heterocyclyl, wobei Heterocyclyl die Bedeutung von

-für eine Aminoschutzgruppe.

$R^2$ -für Wasserstoff.

-für Fluor, Chlor, Brom oder Azido.

-für Phenyl,

-für $NHR^1$. wobei $R^1$ die oben angegebene Bedeutung hat.

-für einen Rest der Formel

oder 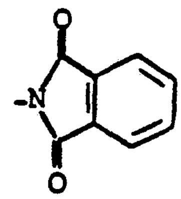 ,

gegebenenfalls durch Methyl, Amino, Hydroxy oder Acetylamino substituiertes Triazolyl, Tetrazolyl, Thiadiazolyl, Furyl, Thienyl, Pyridyl oder Pyrimidyl hat,

-für $-COR^5$, $-CO_2R^5$, $-CONHR^5$, Amidino,

-für $-NR^5R^6$. Ureido, Guanidino.

-für $-OR^5$, $-OCOR^5$, $-OSO_2R^7$, $-OPO(OR^5)_2$.

-für $-SR^5$, $S(O)_nR^7$ mit n = 1, 2, $-SCOR^5$, $-SCONHR^5$,

$-SO_2OR^5$, $-SO_2NHR^5$, -SCN oder

-für $-PO(OR^5)_2$, $-PO(NR^5R^6)_2$,

wobei jeweils

$R^5$,$R^6$ -gleich oder verschieden sind und

-für Wasserstoff,

-für geradkettiges, verzweigtes oder cyclisches, gesättigtes oder ungesättigtes Alkyl mit bis zu 6 C-Atomen steht, das gegebenenfalls durch Phenyl oder ein oder mehrere Fluor substituiert ist

- für gegebenenfalls durch Nitro oder Methoxy substituiertes Phenyl,

-für Furyl, Thienyl oder Pyridyl, oder

-für eine Hydroxy-, Mercapto-, Amino-oder Carboxyschutzgruppe steht,

$R^7$ -die gleiche Bedeutung hat wie $R^5$. $R^6$. jedoch nicht für Wasserstoff oder eine Schutzgruppe steht.

oder

-für geradkettiges. verzweigtes oder cyclisches. gesättigtes oder ungesättigtes Alkyl mit bis zu 6 C-Atomen. das gegebenenfalls substituiert ist durch Phenyl, Fluor. Chlor. Furyl. Thienyl. Pyridyl. Imidazolyl. Triazolyl. Tetrazolyl. Thiadiazolyl oder einen Rest der Formel

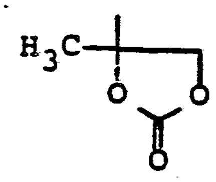 , 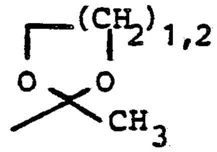 oder 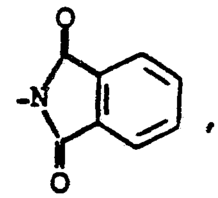 ,

oder durch $-CO_2R^5$,$-CONHR^5$, Cyano. Amidino. $-NHR^5$. $NH-COCH_3$, Guanidino, Azido, $-OR^5$, $-OCOR^5$, $-OSO_2R^7$, $-OPO(OR^5)_2$, $-SR^5$, $-SO_2R^7$, $-SCOR^5$, $-SO_2OR^5$, $-SO_2NHR^5$, $-PO(OR^5)_2$, $-PO(NR^5R^6)_2$, wobei $R^5$, $R^6$, $R^7$ die oben angegebene Bedeutung haben.

und

$R^4$ -für eine Carboxyschutzgruppe oder für einen in vivo spaltbaren Esterrest.

3. Verfahren nach den Ansprüchen 1 und 2. dadurch gekennzeichnet. daß in den Verbindungen der allgemeinen Formeln I. II bzw. III $R^2$. $R^3$. $R^5$. $R^6$ und $R^7$ für geradkettiges. verzweigtes oder cyclisches, gesättigtes oder ungesättigtes Alkyl mit bis zu 10 C-Atomen stehen . das gegebenenfalls substituiert ist durch Reste aus der Gruppe bestehend aus

a) Phenyl. das mehrfach substituiert sein kann durch Methyl, Methoxy, Methylthio, Trifluormethyl, Trifluormethoxy, Trifluormethylthio, Nitro, Cyano, jeweils gegebenenfalls geschütztes Hydroxy, Mercapto oder Amino, Dimethylamino, Carbamoyl, Amidino. Sulfo, Sulfamoyl oder Halogen, bevorzugt Fluor, Chlor oder Brom.

b) gesättigtem oder ungesättigtem 5-6-gliedriges. gegebenenfalls aneinander anelliertes Heterocyclyl mit 1-4 gleichen oder verschiedenen Heteroatomen aus der Gruppe O, S oder N, wobei die Heterocyclen die gleichen Substituenten tragen können wie unter a) für Phenyl angegeben,

c) Halogen. bevorzugt Fluor, Chlor. Brom

d) $-COR^9$, $-CO_2R^9$, $-CO-NR^9R^{10}$, $-C(=NR^{10})NR^9R^{10}$. $-CN$.

e) $-NR^9R^{10}$, $-NR^9-COR^{10}$, $-N(COR^9)$, $(COR^{10})$, $-NR^9-CO_2R^{11}$, $-NR^{10}-CR^9(=NR^{10})$, $-NR^{10}-CO-NR^{10}R^9$, $-NR^9-C(=NR^{10})-NR^9R^{10}$, $-NR^9SO_2R^{11}$, $-N_3$,

f) $-OR^9$, $-OCOR^9$, $-OCO-NR^{10}R^9$, $-OSO_2R^{11}$, $-OSO_3R^9$, $-OPO(OR^{11})OR^9$,

g) $-SR^9$, $S(O)_nR^{11}$ wobei n = 1 oder 2 ist, $-SOCR^{11}$, $-SO_2OR^9$, $-SO_2NR^{10}R^{10}$, oder.

h) $-PX(OR^{10})OR^9$, $PX(NR^{10}R^9)_2$, $PX(OR^9)NR^{10}R^9$, wobei jeweils

$R^9$, $R^{10}$ -gleich oder verschieden sind und

-für Wasserstoff,

-für geradkettiges, verzweigtes oder cyclisches gesättigtes oder ungesättigtes Alkyl mit bis zu 10 C-Atomen steht,

-für Phenyl oder Benzyl,

-für Heterocyclyl oder Heterocyclylmethyl steht, wobei Heterocyclyl die unter b) angegebene Bedeutung hat, oder

-gegebenenfalls für eine Hydroxy-, Mercapto-, Amino-oder Carboxyschutzgruppe steht,

$R^{11}$ -die gleiche Bedeutung hat wie $R^{10}$, $R^9$, jedoch night für Wasserstoff oder eine Schutzgruppe steht,

und

X -für Sauerstoff oder Schwefel steht.

4. Verfahren nach Anspruch 1. dadurch gekennzeichnet. daß $R^2$. $R^3$, $R^5$. $R^6$ und $R^7$ für Phenyl stehen. welches durch $C_1$-$C_4$-Alkyl, $C_1$-$C_4$ Alkylthio, Trifluormethyl. Trifluormethoxy, Trifluormethylthio, Halogen, Cyano, Nitro, Azido, jeweils gegebenenfalls geschütztes Amino, Hydroxy oder Mercapto, Dimethylamino, Sulfo. Sulfamoyl, Acetoxy oder Carbamoyl einfach, zweifach oder dreifach substituiert sein kann.

5. Verfahren nach Anspruch 1, dadurch gekennzeichnet. daß $R^2$,$R^3$, $R^5$, $R^6$ und $R^7$ für gesättigtes oder ungesättigtes, 5-6-gliedriges, gegebenenfalls aneinander anelliertes Heterocyclyl mit 1-4 gleichen oder verschiedenen Heteroatomen aus der Gruppe O, S, N stehen, das ein-, zwei-, oder dreifach substituiert ist durch $C_1$-$C_4$-Alkyl. $C_1$-$C_4$-Alkoxy, $C_1$-$C_4$-Alkylthio, Trifluormethyl, Trifluormethoxy, Trifluormethylthio, Halogen, Cyano, Nitro, Azido, jeweils gegebenenfalls geschütztes Amino, Hydroxy oder Mercapto, Dimethylamino, Sulfo, Sulfamoyl, Acetoxy oder Carbamoyl.

6. Verfahren nach den Ansprüchen 1 bis 5, dadurch gekennzeichnet, daß man die Reaktion in Dimethoxyethan, Diglyme, Triglyme, Tetrahydrofuran, Dioxan, Diethylether, tert. Butylmethylether, Dichlormethan. Trichlormethan.Tetrachlormethan, 1,1,2-Trichlorethan, Dichlorethan, Trichlorethylen, Chlorbenzol, Dichlorbenzol, Essigsäureethylester, Toluol oder Cyclohexan bei Temperaturen von -30°C bis +50°C in Gegenwart von Triethyl-, Tripropyl-oder Tributylamin, Ethyldiisopropylamin, Pyridin, Picolin, Lutidin, N-Methylmorpholin, N-Methylpiperidin, 1,5-Diazabicyclo [5,4,0]undec-5-en (DBU) oder 1,5-Diazabicyclo[4,3,0]non-3-en (DBN) durchführt.

7. Verfahren nach den Ansprüchen 1 bis 6, dadurch gekennzeichnet, daß man als Silylierungsmittel bzw. Katalysator Trialkylsilylperfluoralkansulfonate. Trialkylsilylalkansulfonate, Trialkylsilylarylsulfonate, Trialkylsilylperfluoralkanoate, Trialkylsilylalkanoate, Trialkylsilylhalogenide, Trialkylsilylperchlorate, Trialkylsilyldifluorphosphate, Trialkylsilyldichlorphosphate, Trialkylsilylfluorosulfate. N,O-Bis(trialkylsilyl)-acetamide, Trialkylsilylcyanide einsetzt.

8. Verfahren nach den Ansprüchen 1 bis 7, dadurch gekennzeichnet. daß man als Silylierungsmittel Trimethylsilyltrifluormethansulfonat, Trimethylsilylnonafluorobutansulfonat, Trimethylsilyltrifluoracetat, Trimethylsilylperchlorat, Trimethylsilylbromid, Trimethylsilyldifluorphosphat, Trimethylsilylfluorsulfonat, N,O-Bis(trimethylsilyl)trifluoracetamid verwendet.

9. Verfahren nach den Ansprüchen 1 bis 8. dadurch gekennzeichnet, daß man

a) pro Äquivalent der Verbindung III 0.1-1 Äquivalent der Verbindung II.

b) pro Äquivalent der Verbindung III 1-2 Äquivalente Silylierungsmittel.

c) pro Äquivalent Silylierungsmittel. 1.01-3 Äquivalent Base

einsetzt und nach 0.1-24 h soviel weiteres Silylierungsmittel zugibt. daß dann das Silylierungsmittel in einem geringen Überschuß über die Base vorhanden ist und gegebenenfalls außer den oben angegebenen Mengenverhältnissen an Silylierungsmittel und Base für jede freie. d.h. zu - schützende Hydroxy-, Mercapto-bzw. Amingruppe in den Verbindungen II und III jeweils ein zusätzliches Äquivalent Silylierungsmittel und Base einsetzt.

10. Verbindungen der Formel I. in welcher

$R^1$ -für Wasserstoff oder

-für Aminoschutzgruppe steht.

$R^2$ -für Wasserstoff.

-für Halogen, -für Azido oder Phenyl.

-für $NHR^1$ steht. wobei $R^1$ die oben angegebene Bedeutung hat.

-für einen Rest der Formel

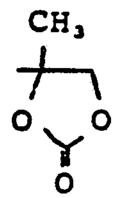

,

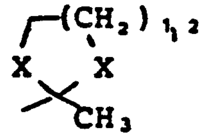

oder

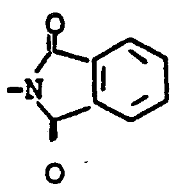

steht,

wobei X Schwefel oder Sauerstoff bedeutet, oder

-für geradkettiges, verzweigtes oder cyclisches, gesättigtes oder ungesättigtes Alkyl mit zu 6 C-Atomen steht, das gegebenenfalls substituiert ist durch Fluor, Chlor oder die Gruppe $O-R^8$,

wobei

$R^8$ -für Wasserstoff,

-für Trimethylsilyl, Triethylsilyl, Triiscpropylsilyl, tert.Butyldimethylsilyl, Triphenylsilyl, Trimethylsilylethoxycarbonyl,

-für Benzyl, Benzyloxycarbonyl, 2-bzw. 4-Nitrobenzyl, 2-bzw. 4-Nitrobenzyloxycarbonyl, tert.-Butyloxycarbonyl, Allyloxycarbonyl, 4-Methoxybenzyl, 4-Methoxybenzyloxycarbonyl oder

-für Formyl, Acetyl, Trichloracetyl, Trichlorethoxycarbonyl, 2,4-Dimethoxybenzyloxycarbonyl, Methoxymethyl, Methylthiomethyl. Methoxyethoxymethyl, [2-(Trimethylsilyl)-ethoxycarbonyl, Tetrahydropyranyl, oder Benzoyl steht,

$R^3$ -für gegebenenfalls durch Chlor, Nitro, Methyl, Methoxy, Methylthio, Trifluormethyl oder gegebenenfalls geschütztes Hydroxy substituiertes Aryl mit 6 bis 10 C-Atomen steht, oder

-für Halogen, Cyano, Azido, Cyanato, Trifluormethyl oder Nitro steht,

-für $-COR^{17}$, $-CONR^{18}R^{19}$, $-CSNR^{18}R^{19}$, oder

-für Allyloxycarbonyl oder 4-Nitrobenzyloxycarbonyl steht,

-für $-NR^{19}R^{18}$, Ureido. Guanidino. Amidino. $-NR^{18}SO_2R^{20}$.

- für $-OSO_2R^{20}$, $-OSO_3R^{18}$ oder $-OPO(OR^{19})OR^{18}$.

-für $-SR^{18}$, $-S(O)_nR^{20}$ mit $n = 1,2$, $-SCOR^{18}$, $-SCONHR^{18}$, $-SO_2OR^{18}$, $-SO_2NR^{19}R^{18}$, -SCN, oder

-für -S-Heterocyclyl steht, wobei Heterocyclyl die Bedeutung von gegebenenfalls durch Methyl, Amino, Hydroxy oder Acetylamino substituiertem Triazolyl, Tetrazolyl oder Thiadiazolyl, Furyl, Thienyl, Pyridyl oder Pyrimidyl hat.

-für $PO(OR^{19})oR^{18}$ oder

-für

- für 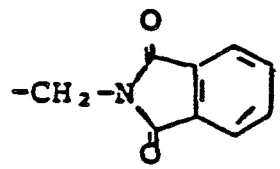 oder Benzyl steht.

oder Benzyl steht.

wobei

$R^{19}$, $R^{18}$ -gleich oder verschieden sind und

-für Wasserstoff.

-für geradkettiges oder verzweigtes Alkyl (bis $C_6$) steht.

-für Phenyl oder Benzyl, (Phenyl gegebenenfalls durch Nitro, Methyl oder Methoxy substituiert). oder

-für eine Hydroxy-, Mercapto-oder Amino schutzgruppe steht und

$R^{20}$ -die gleiche Bedeutung wie $R^{19}$, $R^{18}$ hat, jedoch nicht für Wasserstoff oder eine Schutzgruppe steht,

und

$R^4$ -für Wasserstoff oder

-für Methyl, Ethyl, tert.Butyl. Decyl. 2-Chlorethyl, 2,2,2-Trichlorethyl, Cyano ethyl, Di-bzw, Triphenylmethyl, Acetoxymethyl, Allyl, Benzyl, 4-Methoxybenzyl, 2,4-Dimethoxybenzyl, tert. Butyl-dimethylsilyl, 1-Phenoxyethyl oder 2-Methyl-2-propenyl, 4-Nitrobenzyl, 2-Nitrobenzyl, Trimethylsilylethyl oder Dimethyl-tert.Butylsilylethyl, oder

-für einen Rest der Formel

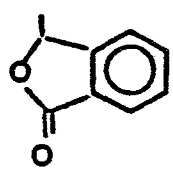 , 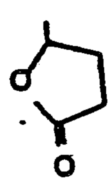 , 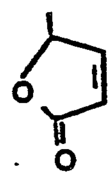 , 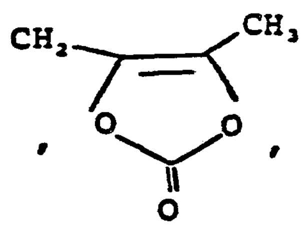 ,

$-CH_2-OCO-C(CH_3)_3$, $-CH(CH_3)OCOOC_2H_5$ oder $-CH_2OCOCH_3$ steht.

11. Verbindungen der Formel I nach Anspruch 10 in welchen

$R^1$ -für Wasserstoff oder

-für eine Aminoschutzgruppe steht,

$R^2$ -für Wasserstoff, Chlor, Brom, Azido, -für $NHR^1$ steht, wobei $R^1$ die oben angegebene Bedeutung hat,

-für einen Rest der Formel

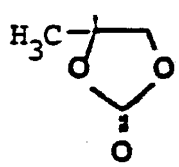 , 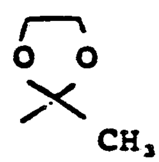 oder

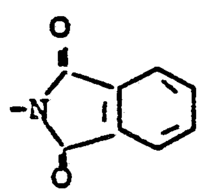 steht, oder

-für Methyl, Ethyl, i-Propyl, tert.Butyl oder

- für 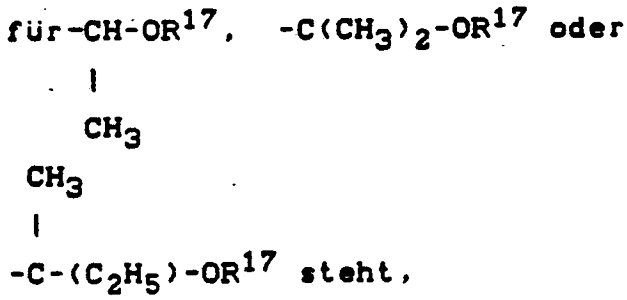 steht,

wobei

$R^{17}$ -für Wasserstoff,

-für Trimethylsilyl, tert. Butyl-dimethylsilyl,

-für Benzyloxycarbonyl, 4-Nitrobenzyloxycarbonyl, tert.Butoxycarbonyl, Allyloxycarbonyl, oder

-für Formyl oder Acetyl steht,

$R^3$ -für Phenyl,

-für Chlor, Brom, Cyano, Cyanato, Azido oder $CF_3$ steht, oder

-für $-COR^{18}$, $-CONR^{19}R^{18}$,

-für Allyloxycarbonyl oder 4-Nitrobenzyloxycarbonyl steht.

-für-$NHR^{18}$. Ureido. Guanidino. Amidino. $-NHSO_2R^{20}$.

-für $-OSO_2R^{20}$. $-OPO(OR^{19})OR^{18}$

-für $-S-R^{18}$. $-SCOR^{18}$. $-SO_2OR^{18}$. $-SO_2NHR^{18}$. -SCN. $-SO_2R^{20}$. $-SCONH_2$ steht oder

-für -S-Pyridyl, -S-Pyrimidyl, -S-Methyltetrazolyl, -S-Thiadiazolyl, 2-Aminothiadiazolyl steht, oder

-für

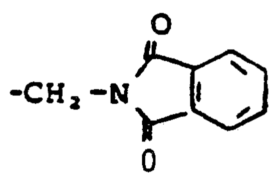

steht.

wobei

$R^{18}$, $R^{19}$ -gleich oder verschieden sind

-für Wasserstoff oder

-für geradkettiges oder verzweigtes Alkyl (bis $C_4$) steht.

-für Phenyl, Benzyl oder 4-Methylphenyl, oder für eine Hydroxy-, Mercapto-oder Aminoschutzgruppe steht.

und $R^{20}$ -die gleiche Bedeutung hat wie $R^{18}$, $R^{19}$ jedoch nicht für Wasserstoff oder eine Schutzgruppe steht,

$R^4$ -für Wasserstoff oder

-für Methyl, Ethyl, tert.Butyl, 2,2,2-Trichlorethyl, Allyl, Acetonyl, 4-Nitrobenzyl, 2-Nitrobenzyl, 4-Methoxybenzyl, Benzyl oder Trimethylsilylethyl oder

-für einen Rest der Formel

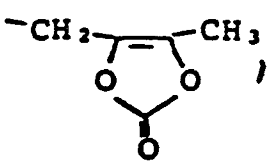 , $-CH(CH_3)OCOOC_2H_5$ oder $CH_2OCOC(CH_3)_3$

steht.

12. Verwendung von Verbindungen der Formel I

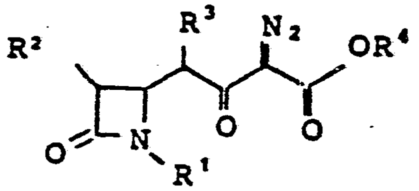

(I),

in welcher

$R^1$ -für Wasserstoff oder

-für eine Aminoschutzgruppe steht,

$R^2$ -für Wasserstoff,

-für gegebenenfalls substituiertes Alkyl,

-für gegebenenfalls substituiertes Aryl,

-für gegebenenfalls substituiertes Heterocyclyl,

-für Cyano, Azido, Halogen, oder

-für $NHR^1$ steht, wobei $R^1$ die oben angegebene Bedeutung hat,

$R^3$ -für Wasserstoff,

-für gegebenenfalls substituiertes Alkyl,

-für gegebenenfalls substituiertes Aryl,

-für gegebenenfalls substituiertes Heterocyclyl,

-für Halogen, Cyano, Cyanato, Azido, Nitro oder =N-Phenyl,

- für $-COR^5$, $-CO_2R^5$, $-CO-NR^5R^6$, $-CSNR^5R^6$, $-C(=NR^5)-NR^5R^6$,

-für $-NR^5R^6$, $-NR^5-COR^6$, $-NR^5-CO_2R^7$, $-NR^6-CR^5(=NR^6)$, $-NR^6-CO-NR^5R^6$, $-NR^5-CS-NR^5R^6$, $-NR^5C(=NR^6)-NR^5R^6$, $-NR^5-SO_2R^7$, oder

-für $OR^5$, $-OCOR^5$, $-OCO-NR^5R^6$. $-OSO_2R^7$, $OSO_3R^5$, $-OPO(OR^5)OR^6$, oder

-für $-SR^5$, $-S(O)_nR^7$ wobei n=1 oder 2 ist, $-SCOR^5$, $-SO_2OR^5$, $-SO_2NR^5R^6$, -SCN, $-SCONHR^5$, -für $-PX(OR^5)-OR^6$, $PX(NH^5R^6)_2$, $PX(OR^5)NR^5R^6$

wobei jeweils

$R^5$ ,$R^6$ -gleich oder verschieden sind und

-für gegebenenfalls substituiertes Alkyl,

-für gegebenenfalls substituiertes Aryl,

-für gegebenenfalls substituiertes Heterocyclyl steht, oder

-gegebenenfalls für eine Hydroxy-, Mercapto-, Amino-oder Carboxyschutzgruppe steht,

$R^7$ -die gleiche Bedeutung hat wie $R^5$, $R^6$, jedoch nicht für Wasserstoff oder eine Schutzgruppe steht,

und

X -für Sauerstoff oder Schwefel steht

und

$R^4$ -für eine Carboxyschutzgruppe oder

-für einen in vivo spaltbaren Esterest steht,

hergestellt nach dam Verfahren gemäß Anspruch 1, zur Herstellung von Carbapenemantibiotika.

13. Verwendung von Verbindungen der Formel I

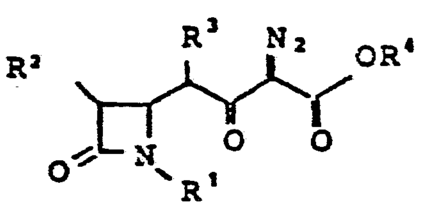

(I),

in welcher

$R^1$ -für Wasserstoff oder

-für eine Aminoschutzgruppe steht,

$R^2$ -für Wasserstoff.

-für gegebenenfalls substituiertes Alkyl,

-für gegebenenfalls substituiertes Aryl,

-für gegebenenfalls substituiertes Heterocyclyl,

-für Cyano, Azido. Halogen. oder -für $NHR^1$ steht. wobei $R^1$ die oben angegebene Bedeutung hat,

$R^3$ -für Wasserstoff.

-für gegebenenfalls substituiertes Alkyl.

-für gegebenenfalls substituiertes Aryl.

-für gegebenenfalls substituiertes Heterocyclyl.

-für Halogen, Cyano, Cyanato, Azido, Nitro oder =N-Phenyl,

-für $-COR^5$. $-CO_2R^5$. $-CO-NR^5R^6$. $-CSNR^5R^6$. $-C(=NR^5)-NR^5R^6$.

-für $-NR^5R^6$. $-NR^5-COR^6$, $-NR^5-CO_2R^7$, $-NR^6-CR^5(=NR^6)$, $-NR^6-CO-NR^5R^6$, $-NR^5-CS-NR^5R^6$, $-NR^5-C(=NR^6)-NR^5NR^6$, $-NR^5-SO_2R^7$, oder

-für$OR^5$, $-OCOR^5$, $-OCO-NR^5R^6$. $-OSO_2R^7$, $-OSO_3R^5$, $-OPO(OR^5)OR^6$, oder

-für $-SR^5$, $-S(O)_nR^7$ wobei n=1 oder 2 ist, $-SCOR^5$, $-SO_2OR^5$, $-SO_2NR^5R^6$, -SCN, $-SCONHR^5$,

-für $-PX(OR^5)OR^6$, $PX(NH^5R^6)_2$, $PX(OR^5)NR^5R^6$, wobei jeweils

$R^5$, $R^6$ -gleich oder verschieden sind und

-für gegebenenfalls substituiertes Alkyl,

-für gegebenenfalls substituiertes Aryl,

-für gegebenenfalls substituiertes Heterocyclyl steht, oder

-gegebenenfalls für eine Hydroxy-, Mercapto-, Amino-oder Carboxyschutzgruppe steht,

$R^7$ -die gleiche Bedeutung hat wie $R^5$, $R^6$, jedoch nicht für Wasserstoff oder eine Schutzgruppe steht,

und

X -für Schwefel oder Sauerstoff steht,

und

$R^4$ -für eine Carboxyschutzgruppe oder

-für einen in vivo spaltbaren Esterrest steht,

zur Herstellung von Carbapenamantibiotika.

Europäisches Patentamt

# EUROPÄISCHER RECHERCHENBERICHT

Nummer der Anmeldung

EP 86 10 2971

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CANADIAN JOURNAL OF CHEMISTRY, Band 62, Nr. 12, 1984, Seiten 2936-2940, Canada; YASUTSUGU UEDA et al.: "A simple method of preparing trimethylsilyl- and tert-butyldimethylsilyl-enol ethers of alpha-diazoacetoacetates and their use in the synthesis of a chiral precursor to thienamycin analogs" --- | 1,10, 12 | C 07 D 205/08<br>C 07 F 7/18<br>C 07 F 9/65<br>C 07 D 403/12<br>C 07 D 487/04 //<br>C 07 C 119/00<br>C 07 C 154/00<br>(C 07 D 487/04<br>C 07 D 209:00<br>C 07 D 205:00 ) |
| D,Y | EP-A-0 078 026 (MERCK)<br>* Seiten 8-11; Ansprüche *<br>----- | 1,10, 12 | |
| | | | RECHERCHIERTE SACHGEBIETE (Int. Cl.4) |
| | | | C 07 D 205/00<br>C 07 D 403/00<br>C 07 D 487/00 |

Der vorliegende Recherchenbericht wurde für alle Patentansprüche erstellt.

| Recherchenort | Abschlußdatum der Recherche | Prüfer |
|---|---|---|
| DEN HAAG | 18-06-1986 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82